# EUROPEAN PATENT SPECIFICATION

(11) **EP 3 292 139 B1**
(45) Date of publication and mention of the grant of the patent: **28.10.2020**
(21) Application number: 16790043.0
(22) Date of filing: 04.05.2016
(51) Int. Cl.: C07K 14/47, C07K 16/18, G01N 33/574, A61P 35/00, C12N 5/0783, G01N 33/50

(54) **H3.3 CTL PEPTIDES AND USES THEREOF**
H3.3-CTL-PEPTIDE UND VERWENDUNGEN DAVON
PEPTIDES CTL H3.3 ET LEURS UTILISATIONS

(30) Priority: 05.05.2015 US 201562157362 P; 31.08.2015 US 201562212508 P
(43) Date of publication of application: 14.03.2018
(60) Divisional application: 20197179.3
(73) Proprietor: The Regents of the University of California, Oakland, CA 94607 (US)
(72) Inventor: OKADA, Hideo, Oakland, California 94607 (US); HOU, Yafei, Oakland, California 94607 (US)
(74) Representative: J A Kemp LLP
(86) International application number: PCT/US2016/030849
(87) International publication number: WO 2016/179326

(56) References cited:
- WO-A1-2013/075237
- WO-A2-2009/147201
- US-A1- 2013 236 906
- US-A1- 2014 107 039
- US-A1- 2014 107 039
- P. W. LEWIS ET AL: "Inhibition of PRC2 Activity by a Gain-of-Function H3 Mutation Found in Pediatric Glioblastoma", SCIENCE, vol. 340, no. 6134, 17 May 2013 (2013-05-17), pages 857-861, XP055253687, ISSN: 0036-8075, DOI: 10.1126/science.1232245
- CUTHBERT G L ET AL: "Histone deimination antagonizes arginine methylation", CELL, CELL PRESS, AMSTERDAM, NL, vol. 118, no. 5, 3 September 2004 (2004-09-03), pages 545-553, XP002358960, ISSN: 0092-8674, DOI: 10.1016/J.CELL.2004.08.020
- KOVALIC D K ET AL: "Wheat recombinant protein SEQ ID NO 152042", GENESEQ,, 22 February 2007 (2007-02-22), XP002751291, [retrieved on 2011-02-03] & US 2007/044171 A1 (KOVALIC DAVID K [US] ET AL) 22 February 2007 (2007-02-22)
- DATABASE Geneseq [Online] 10 May 2012 (2012-05-10), "Cytomegalovirus phosphoprotein 65 specific TCR alpha chain, SEQ ID 10.", XP002786132, retrieved from EBI accession no. GSP:AZU40581 Database accession no. AZU40581 -& WO 2012/038055 A1 (UNICELL GMBH [DE]; UNIVERSITAETSMEDIZIN DER JOHANNES GUTENBERG UNI MAI) 29 March 2012 (2012-03-29)
- M. I. VANAN ET AL: "Management of high-grade gliomas in the pediatric patient: Past, present, and future", NEURO-ONCOLOGY PRACTICE, vol. 1, no. 4, 12 September 2014 (2014-09-12), pages 145-157, XP55519137, ISSN: 2054-2577, DOI: 10.1093/nop/npu022
- T Fukazawa ET AL: "Testing the importance of each residue in a HLA-B27-binding peptide using monoclonal antibodies", The Journal of Immunology, 1 February 1994 (1994-02-01), pages 1190-1196, XP55518741, United States Retrieved from the Internet: URL:http://www.jimmunol.org/content/152/3/ 1190.full-text.pdf
- YAFEI HOU ET AL: "Novel and shared neoantigen for glioma T cell therapy derived from histone 3 variant H3.3 K27M mutation", JOURNAL FOR IMMUNOTHERAPY OF CANCER, BIOMED CENTRAL LTD, LONDON, UK, vol. 3, no. 2, 4 November 2015 (2015-11-04), pages 1-2, XP021235622, DOI: 10.1186/2051-1426-3-S2-P445
- CUTHBERT ET AL.: 'Histone deimination antagonizes arginine methylation.' CELL vol. 118, no. 5, 03 September 2004, pages 545 - 553, XP002358960

### SEQUENCE LISTING

<110> The Regents of the University of California
   Okada, Hideho
   Hou, Yafei
<120> H3.3 CTL PEPTIDES AND USES THEREOF
<130> 1006640
<150> US 62/157,362
   <151> 2015-05-05
<150> US 62/212,508
   <151> 2015-08-31
<160> 17
<170> PatentIn version 3.5
<210> 1
   <211> 10
   <212> PRT
   <213> Artificial Sequence
<220>
   <223> synthetic peptide construct
<220>
   <221> MOD_RES
   <222> (1)..(1)
   <223> Xaa is Arg or Ala
<220>
   <221> MOD_RES
   <222> (1)..(1)
   <223> Xaa is Arg or Ala; when Xaa is Arg, citrullination of Arg may be present or absent
<220>
   <221> MOD_RES
   <222> (6)..(6)
   <223> Xaa is Ser or Ala
<400> 1
   Xaa Met Ser Ala Pro Xaa Thr Gly Gly Val
   1               5                   10
<210> 2
   <211> 10
   <212> PRT
   <213> Artificial Sequence
<220>
   <223> synthetic peptide construct
<400> 2
   Arg Met Ser Ala Pro Ser Thr Gly Gly Val
   1               5                   10
<210> 3
   <211> 10
   <212> PRT
   <213> Artificial Sequence
<220>
   <223> synthetic peptide construct
<400> 3
   Arg Lys Ser Ala Pro Ser Thr Gly Gly Val
   1               5                   10
<210> 4
   <211> 10
   <212> PRT
   <213> Artificial Sequence
<220>
   <223> synthetic peptide construct
<400> 4
   Arg Met Ser Ala Pro Ser Thr Gly Gly Val
   1               5                   10
<210> 5
   <211> 10
   <212> PRT
   <213> Artificial Sequence
<220>
   <223> synthetic peptide construct
<400> 5
   Ala Met Ser Ala Pro Ser Thr Gly Gly Val
   1               5                   10
<210> 6
   <211> 10
   <212> PRT
   <213> Artificial Sequence
<220>
   <223> synthetic peptide construct
<400> 6
   Arg Met Ser Ala Pro Ala Thr Gly Gly Val
   1               5                   10
<210> 7
   <211> 10
   <212> PRT
   <213> Artificial Sequence
<220>
   <223> synthetic peptide construct
<400> 7
   Ala Met Ser Ala Pro Ala Thr Gly Gly Val
   1               5                   10
<210> 8
   <211> 273
   <212> PRT
   <213> Artificial Sequence
<220>
   <223> synthetic peptide construct
<400> 8
   Met Leu Thr Ala Ser Leu Leu Arg Ala Val Ile Ala Ser Ile Cys Val
   1               5                   10                  15
   Val Ser Ser Met Ala Gln Lys Val Thr Gln Ala Gln Thr Glu Ile Ser
               20                  25                  30
   Val Val Glu Lys Glu Asp Val Thr Leu Asp Cys Val Tyr Glu Thr Arg
           35                  40                  45
   Asp Thr Thr Tyr Tyr Leu Phe Trp Tyr Lys Gln Pro Pro Ser Gly Glu
       50                  55                  60
   Leu Val Phe Leu Ile Arg Arg Asn Ser Phe Asp Glu Gln Asn Glu Ile
   65                  70                  75                  80
   Ser Gly Arg Tyr Ser Trp Asn Phe Gln Lys Ser Thr Ser Ser Phe Asn
                   85                  90                  95
   Phe Thr Ile Thr Ala Ser Gln Val Val Asp Ser Ala Val Tyr Phe Cys
               100                 105                 110
   Ala Leu Ser Glu Glu Asn Asp Met Arg Phe Gly Ala Gly Thr Arg Leu
           115                 120                 125
   Thr Val Lys Pro Asn Ile Gln Asn Pro Asp Pro Ala Val Tyr Gln Leu
       130                 135                 140
   Arg Asp Ser Lys Ser Ser Asp Lys Ser Val Cys Leu Phe Thr Asp Phe
   145                 150                 155                 160
   Asp Ser Gln Thr Asn Val Ser Gln Ser Lys Asp Ser Asp Val Tyr Ile
                   165                 170                 175
   Thr Asp Lys Thr Val Leu Asp Met Arg Ser Met Asp Phe Lys Ser Asn
               180                 185                 190
   Ser Ala Val Ala Trp Ser Asn Lys Ser Asp Phe Ala Cys Ala Asn Ala
           195                 200                 205
   Phe Asn Asn Ser Ile Ile Pro Glu Asp Thr Phe Phe Pro Ser Pro Glu
       210                 215                 220
   Ser Ser Cys Asp Val Lys Leu Val Glu Lys Ser Phe Glu Thr Asp Thr
   225                 230                 235                 240
   Asn Leu Asn Phe Gln Asn Leu Ser Val Ile Gly Phe Arg Ile Leu Leu
                   245                 250                 255
   Leu Lys Val Ala Gly Phe Asn Leu Leu Met Thr Leu Arg Leu Trp Ser
               260                 265                 270
   Ser
<210> 9
   <211> 822
   <212> DNA
   <213> Artificial Sequence
<220>
   <223> synthetic nucleotide sequence
<400> 9
   atgctgactg ccagcctgtt gagggcagtc atagcctcca tctgtgttgt atccagcatg        60
   gctcagaagg taactcaagc gcagactgaa atttctgtgg tggagaagga ggatgtgacc       120
   ttggactgtg tgtatgaaac ccgtgatact acttattact tattctggta caagcaacca       180
   ccaagtggag aattggtttt ccttattcgt cggaactctt ttgatgagca aaatgaaata       240
   agtggtcggt attcttggaa cttccagaaa tccaccagtt ccttcaactt caccatcaca       300
   gcctcacaag tcgtggactc agcagtatac ttctgtgctc tgagtgagga gaatgacatg       360
   cgctttggag cagggaccag actgacagta aaaccaaata tccagaaccc tgaccctgcc       420
   gtgtaccagc tgagagactc taaatccagt gacaagtctg tctgcctatt caccgatttt       480
   gattctcaaa caaatgtgtc acaaagtaag gattctgatg tgtatatcac agacaaaact       540
   gtgctagaca tgaggtctat ggacttcaag agcaacagtg ctgtggcctg gagcaacaaa       600
   tctgactttg catgtgcaaa cgccttcaac aacagcatta ttccagaaga caccttcttc       660
   cccagcccag aaagttcctg tgatgtcaag ctggtcgaga aaagctttga aacagatacg       720
   aacctaaact ttcaaaacct gtcagtgatt gggttccgaa tcctcctcct gaaagtggcc       780
   gggtttaatc tgctcatgac gctgcggctg tggtccagct ga                          822
<210> 10
   <211> 311
   <212> PRT
   <213> Artificial Sequence
<220>
   <223> synthetic peptide construct
<400> 10
   Met Gly Pro Gln Leu Leu Gly Tyr Val Val Leu Cys Leu Leu Gly Ala
   1               5                   10                  15
   Gly Pro Leu Glu Ala Gln Val Thr Gln Asn Pro Arg Tyr Leu Ile Thr
               20                  25                  30
   Val Thr Gly Lys Lys Leu Thr Val Thr Cys Ser Gln Asn Met Asn His
           35                  40                  45
   Glu Tyr Met Ser Trp Tyr Arg Gln Asp Pro Gly Leu Gly Leu Arg Gln
       50                  55                  60
   Ile Tyr Tyr Ser Met Asn Val Glu Val Thr Asp Lys Gly Asp Val Pro
   65                  70                  75                  80
   Glu Gly Tyr Lys Val Ser Arg Lys Glu Lys Arg Asn Phe Pro Leu Ile
                   85                  90                  95
   Leu Glu Ser Pro Asn Pro Asn Gln Thr Ser Leu Tyr Phe Cys Ala Ser
               100                 105                 110
   Gly Trp Gly Gly Pro Phe Tyr Glu Gln Tyr Phe Gly Pro Gly Thr Arg
           115                 120                 125
   Leu Thr Val Thr Glu Asp Leu Lys Asn Val Phe Pro Pro Glu Val Ala
       130                 135                 140
   Val Phe Glu Pro Ser Glu Ala Glu Ile Ser His Thr Gln Lys Ala Thr
   145                 150                 155                 160
   Leu Val Cys Leu Ala Thr Gly Phe Tyr Pro Asp His Val Glu Leu Ser
                   165                 170                 175
   Trp Trp Val Asn Gly Lys Glu Val His Ser Gly Val Ser Thr Asp Pro
               180                 185                 190
   Gln Pro Leu Lys Glu Gln Pro Ala Leu Asn Asp Ser Arg Tyr Cys Leu
           195                 200                 205
   Ser Ser Arg Leu Arg Val Ser Ala Thr Phe Trp Gln Asn Pro Arg Asn
       210                 215                 220
   His Phe Arg Cys Gln Val Gln Phe Tyr Gly Leu Ser Glu Asn Asp Glu
   225                 230                 235                 240
   Trp Thr Gln Asp Arg Ala Lys Pro Val Thr Gln Ile Val Ser Ala Glu
                   245                 250                 255
   Ala Trp Gly Arg Ala Asp Cys Gly Phe Thr Ser Glu Ser Tyr Gln Gln
               260                 265                 270
   Gly Val Leu Ser Ala Thr Ile Leu Tyr Glu Ile Leu Leu Gly Lys Ala
           275                 280                 285
   Thr Leu Tyr Ala Val Leu Val Ser Ala Leu Val Leu Met Ala Met Val
       290                 295                 300
   Lys Arg Lys Asp Ser Arg Gly
   305                 310
<210> 11
   <211> 936
   <212> DNA
   <213> Artificial Sequence
<220>
   <223> synthetic nucleotide sequence
<400> 11
   atgggccccc agctccttgg ctatgtggtc ctttgccttc taggagcagg ccccctggaa      60
   gcccaagtga cccagaaccc aagatacctc atcacagtga ctggaaagaa gttaacagtg     120
   acttgttctc agaatatgaa ccatgagtat atgtcctggt atcgacaaga cccagggctg     180
   ggcttaaggc agatctacta ttcaatgaat gttgaggtga ctgataaggg agatgttcct     240
   gaagggtaca aagtctctcg aaaagagaag aggaatttcc ccctgatcct ggagtcgccc     300
   aaccccaacc agacctctct gtacttctgt gccagcggct ggggtggtcc attctacgag     360
   cagtacttcg ggccgggcac caggctcacg gtcacagagg acctgaaaaa cgtgttccca     420
   cccgaggtcg ctgtgtttga gccatcagaa gcagagatct cccacaccca aaaggccaca     480
   ctggtatgcc tggccacagg cttctacccc gaccacgtgg agctgagctg gtgggtgaat     540
   gggaaggagg tgcacagtgg ggtcagcaca gacccgcagc ccctcaagga gcagcccgcc     600
   ctcaatgact ccagatactg cctgagcagc cgcctgaggg tctcggccac cttctggcag     660
   aacccccgca accacttccg ctgtcaagtc cagttctacg ggctctcgga gaatgacgag     720
   tggacccagg atagggccaa acccgtcacc cagatcgtca gcgccgaggc ctggggtaga 780
   gcagactgtg gcttcacctc cgagtcttac cagcaagggg tcctgtctgc caccatcctc 840
   tatgagatct tgctagggaa ggccaccttg tatgccgtgc tggtcagtgc cctcgtgctg 900
   atggccatgg tcaagagaaa ggattccaga ggctag 936
<210> 12
   <211> 7
   <212> PRT
   <213> Artificial Sequence
<220>
   <223> synthetic peptide construct
<400> 12
   Thr Arg Asp Thr Thr Tyr Tyr
   1               5
<210> 13
   <211> 4
   <212> PRT
   <213> Artificial Sequence
<220>
   <223> synthetic peptide construct
<400> 13
   Arg Asn Ser Phe
   1
<210> 14
   <211> 4
   <212> PRT
   <213> Artificial Sequence
<220>
   <223> synthetic peptide construct
<400> 14
   Ala Leu Ser Glu
   1
<210> 15
   <211> 6
   <212> PRT
   <213> Artificial Sequence
<220>
   <223> synthetic peptide construct
<400> 15
   Asn Met Asn His Glu Tyr
   1 5
<210> 16
   <211> 8
   <212> PRT
   <213> Artificial Sequence
<220>
   <223> synthetic peptide construct
<400> 16
   Tyr Ser Met Asn Val Glu Val Thr
   1 5
<210> 17
   <211> 13
   <212> PRT
   <213> Artificial Sequence
<220>
   <223> synthetic peptide construct
<400> 17
   Cys Ala Ser Gly Trp Gly Gly Pro Phe Tyr Glu Gln Tyr
   1 5 10

## Description

This invention was made with government support under grant no. R21 NS083171 awarded by the National Institutes of Health. The government has certain rights in the invention.

### BACKGROUND OF THE INVENTION

Malignant gliomas, including glioblastoma (GBM) and diffuse intrinsic pontine gliomas (DIPG), are lethal brain tumors in both adults and children. Recent genetic studies have revealed that malignant gliomas in children and young adults often show recurrent missense mutations in *H3F3A,* which encodes the replication-independent histone 3 variant H3.3. *See, e.g.,* Lewis et al., Science Vol. 340 no. 6134 pp. 857-861 (2013). Approximately 30 % of overall GBM and 70% of DIPG cases harbor the amino-acid substitution from lysine (K) to methionine (M) at the position 27 of H3.3 (K27M mutation, hereafter), which is universally associated with shorter survival in DIPG patients compared with patients with non-mutated H3.3. The adaptive immune system, such as T lymphocytes (T cells hereafter), are normally tolerant to normal self-proteins, but can recognize mutated amino-acids as non-self. Hence cancer-specific mutations can be suitable targets of cancer immunotherapy, such as cancer vaccines and adoptive T cell transfer therapy.

US 2014/107039 describes Polycomb Repressive Complex 2 (prc2) inhibitors and uses thereof. WO 2013/075237 describes mutations of histone proteins associated with proliferative disorders. P. W. Lewis et al., Science, 2013, 340:857-861 describe inhibition of PRC2 activity by a gain-of-function H3 mutation found in pediatric glioblastoma. Cuthbert G. L. et al., Cell, 2004, 118(5):545-553 describe that histone deamination antagonizes arginine methylation. WO 2009/147201 describes specific binding molecules directed against citrulline-containing epitopes for use in the therapy and prevention of inflammatory conditions.

### BRIEF SUMMARY OF THE INVENTION

The present invention provides:
(i) A T-cell expressing one or more polypeptides comprising a T-cell receptor (TCR) or fragment thereof that binds to a peptide/major histocompatibility complex (MHC) complex, wherein the MHC is HLA-A*0201, and wherein the peptide in the peptide/MHC complex consists of 10-14 amino acids and comprises RMSAPSTGGV (SEQ ID NO:2).
(ii) A method of enriching for T-cells expressing a TCR that binds to a peptide/MHC complex, wherein the MHC is HLA-A*0201, wherein the peptide in the peptide/MHC complex consists of 10-14 amino acids, and wherein the peptide comprises RMSAPSTGGV (SEQ ID NO:2), the method comprising
   generating a starting culture of T-cells expressing TCRs;
   culturing the T-cells in the presence of the peptide to generate an enriched culture of T-cells, wherein the enriched culture is enriched for T-cells expressing TCRs that bind the peptide/MHC complex compared to the starting culture.
(iii) An isolated peptide consisting of RMSAPSTGGV (SEQ ID NO:2), optionally wherein R in SEQ ID NO:2 is citrullinated or not citrullinated.
(iv) A composition for stimulating an immune response to replication-independent histone 3 variant H3.3, the composition comprising the peptide of (iii), optionally further comprising an adjuvant.
(v) A fluorochrome-conjugated peptide-major histocompatibility complex (pMHC) multimer, wherein the MHC is HLA-A*0201, and wherein the peptide is the peptide of (iii).
(vi) A nucleic acid encoding the peptide of (iii).
(vii) An antigen presenting cell (APC) comprising a heterologous peptide consisting of 10-12 amino acids, wherein the peptide comprises RMSAPSTGGV (SEQ ID NO:2).
(viii) An APC of (vii) for use in a method of treating glioma in a human individual, the method comprising, administering a sufficient amount of the APC to the human individual.
(ix) A composition comprising a peptide consisting of 10-12 amino acids, wherein the peptide comprises RMSAPSTGGV (SEQ ID NO:2), for use in a method of treating glioma in a human individual, the method comprising administering a sufficient amount of the composition to the human individual.

In some aspects, an isolated peptide consisting of less than 100, 75, 50, or 30 amino acids is described, wherein the peptide comprises (R/A)MSAP(S/A)TGGV (SEQ ID NO: 1). In some aspects, the peptide consists of 10-14 amino acids. In some aspects, the peptide consists of (R/A)MSAP(S/A)TGGV (SEQ ID NO: 1). In some aspects, the R in SEQ ID NO: 1 is citrullinated. In some aspects, the R in SEQ ID NO: 1 is not citrullinated.

Also described is a fluorochrome-conjugated peptide-major histocompatibility complex (pMHC) multimer, wherein the peptide is as described above or elsewhere herein.

Also described is a nucleic acid, optionally isolated or purified, encoding the peptide as described above or elsewhere herein. In some aspects, the nucleic acid is a plasmid or a viral vector. In some aspects, the vector is capable of delivering the nucleic acid into an antigen presenting cell (APC).

Also described is a cell comprising a heterologous peptide consisting of 10-12 or 10-14 (e.g., 10, 11, 12, 13, or 14) amino acids, wherein the peptide comprises (R/A)MSAP(S/A)TGGV (SEQ ID NO:1). In some aspects, the peptide consists of (R/A)MSAP(S/A)TGGV (SEQ ID NO:1). In some aspects, the R in SEQ ID NO:1 is citrullinated. In some aspects, the R in SEQ ID NO:1 is not citrullinated. In some aspects, the cell is an antigen presenting cell (APC). In some aspects, the cell is a bacterial cell. In some aspects, the bacterial cell is *E. coli* or *Listeria monocytogenes.* In some aspects, the APC presents the heterologous peptide on the surface of the cell. In some aspects, the APC comprises a heterologous expression cassette comprising a promoter operably linked to a polynucleotide encoding the peptide.

Also described is a method of inducing an immune response in a human individual. In some aspects, the method comprises administering a sufficient amount of the APC as described above or elsewhere herein to the human individual, thereby inducing an immune response in the human individual to replication-independent histone 3 variant H3.3 or H3.1. In some aspects, the APC is from the individual (autologous). In some aspects, the immune response is a cytotoxic T-cell response. In some aspects, the human individual has a glioma. In some aspects, the individual carries an HLA-2*201 allele.

Also described is a composition for stimulating an immune response to replication-independent histone 3 variant H3.3, the composition comprises, a peptide consisting of 10-12 or 10-14 (e.g., 10, 11, 12, 13, or 14) amino acids, wherein the peptide comprises (R/A)MSAP(S/A)TGGV (SEQ ID NO:1). In some aspects, the composition comprises a peptide consisting of (R/A)MSAP(S/A)TGGV (SEQ ID NO:1). In some aspects, the composition further comprises an adjuvant. In some aspects, the R in SEQ ID NO:1 is citrullinated. In some aspects, the R in SEQ ID NO:1 is not citrullinated.

Also described is a method of inducing an immune response in a human individual, the method comprising administering a sufficient amount of the composition as described above or elsewhere herein to the human individual, thereby inducing an immune response in the human individual to histone 3 variant H3.3 or H3.1. In some aspects, the composition comprises an adjuvant selected from the group consisting of polyICLC and Bacillus Calmette-Guerin (BCG) vaccine. In some aspects, the immune response is a cytotoxic T-cell response. In some aspects, the human individual has a glioma In some aspects, the individual carries an HLA-2*201 allele.

Also described is an antibody that specifically binds to RMSAPSTGGV (SEQ ID NO:2) and does not bind to RKSAPSTGGV (SEQ ID NO:3). In some aspects, the antibody is linked to a heterologous detectable label. In some aspects, the label is fluorescent.

Also described is a T-cell expressing a polypeptide or polypeptides comprising a T-cell receptor (TCR), or a peptide/MHC complex binding fragment thereof, that binds the peptide as described above or elsewhere herein in a peptide/MHC complex. In some aspects, the TCR is heterologous to the T-cell. In some aspects, expression of the TCR is under the control of a heterologous promoter (e.g., as transgenes). In some aspects, the TCR comprises one or more of the CDRs as listed in SEQ ID NOs: 12-17, optionally in a heterologous framework region. The TCR will in general be formed of an alpha and a beta chain (two separate polypeptides). In some aspects, the TCR comprises SEQ ID NO:8, SEQ ID NO: 10, or both, either as a fusion protein or as separate proteins.

Also described is a method of enriching for T-cells expressing a TCR that binds the peptide as described above or elsewhere herein. In some aspects, the method comprises generating a starting culture of T-cells expressing TCRs; culturing the T-cells in the presence of the peptide to generate an enriched culture of T-cells, wherein the enriched culture is enriched for T-cells expressing TCRs that bind the peptide compared to the starting culture. In some aspects, the culturing comprises culturing the T-cells in the presence of IL-2, IL-4, IL-7, IL-15, or combinations thereof. In some aspects, the method further comprises sorting cells in the enriched culture for T-cells that bind the peptide in a peptide/MHC complex to form a further enriched population of T-cells expressing TCRs that bind the peptide/MHC complex. In some aspects, the sorting comprises contacting cells in the enriched culture with a fluorochrome-conjugated peptide-major histocompatibility complex (pMHC) multimer.

### DEFINITIONS

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)).

The terms "polypeptide," "peptide" and "protein" are used interchangeably herein to refer to a polymer of amino acid residues. The terms encompass amino acid polymers in which one or more amino acid residue is an artificial chemical mimetic of a corresponding naturally occurring amino acid, as well as naturally occurring amino acid polymers and non-naturally occurring amino acid polymers.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. The term "amino acid analogs" refers to compounds that have the same basic chemical structure as a naturally occurring amino acid, *i.e.,* an α carbon that is bound to a hydrogen, a carboxyl group, an amino group, and an R group, *e.g.,* homoserine, norleucine, methionine sulfoxide, methionine methyl sulfonium. Such analogs have modified R groups (*e.g.*, norleucine) or modified peptide backbones, but retain the same basic chemical structure as a naturally occurring amino acid. The term "amino acid mimetics" refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and UGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

For amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (*see, e.g.,* Creighton, Proteins (1984)).

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (*e.g.*, as commonly used in an ELISA), biotin, digoxigenin, or haptens and proteins which can be made detectable, *e.g.,* by incorporating a radiolabel into the peptide or used to detect antibodies specifically reactive with the peptide.

An antibody can consist of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. An "antibody" functions as a binding protein and is structurally defined as comprising an amino acid sequence from or derived from the framework region of an immunoglobulin-encoding gene of a vertebrate animal.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

The term "antibody" as used herein encompasses antibody fragments that retain antigen-binding specificity. For example, there are a number of well characterized antibody fragments. Thus, for example, pepsin digests an antibody C-terminal to the disulfide linkages in the hinge region to produce F(ab)'2, a dimer of Fab which itself is a light chain joined to VH-CH1 by a disulfide bond. The F(ab)'2 may be reduced under mild conditions to break the disulfide linkage in the hinge region thereby converting the (Fab')2 dimer into an Fab' monomer. The Fab' monomer is essentially an Fab with part of the hinge region (see, *e.g.,* Fundamental Immunology, W.E. Paul, ed., Raven Press, N.Y. (1993), for a more detailed description of other antibody fragments). While various antibody fragments are defined in terms of the digestion of an intact antibody, one of skill will appreciate that fragments can be synthesized de novo either chemically or by utilizing recombinant DNA methodology. Thus, the term antibody, as used herein also includes antibody fragments either produced by the modification or digestion of whole antibodies or synthesized using recombinant DNA methodologies.

Antibodies can include V_{H}-V_{L} dimers, including single chain antibodies (antibodies that exist as a single polypeptide chain), such as single chain Fv antibodies (sFv or scFv) in which a variable heavy and a variable light region are joined together (directly or through a peptide linker) to form a continuous polypeptide. The single chain Fv antibody is a covalently linked V_{H}-V_{L} which may be expressed from a nucleic acid including V_{H}- and V_{L}- encoding sequences either joined directly or joined by a peptide-encoding linker (*e.g.,* Huston, et al. Proc. Nat. Acad. Sci. USA, 85:5879-5883, 1988). While the V_{H} and V_{L} are connected to each as a single polypeptide chain, the V_{H} and V_{L} domains associate non-covalently. Alternatively, the antibody can be another fragment. Other fragments can also be generated, *e.g.,* using recombinant techniques, as soluble proteins or as fragments obtained from display methods. Antibodies can also include diantibodies and miniantibodies. Antibodies described herein also include heavy chain dimers, such as antibodies from camelids. Thus, in some aspects an antibody is dimeric. In other aspects, the antibody may be in a monomeric form that has an active isotype. In some aspects the antibody is in a multivalent form, *e.g.,* a trivalent or tetravalent form.

As used herein, "complementarity-determining region (CDR)" refers to the three hypervariable regions in each chain that interrupt the four "framework" regions established by the light and heavy chain variable regions. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found.

As used herein, "V-region" refers to an antibody variable region domain comprising the segments of Framework 1, CDR1, Framework 2, CDR2, and Framework3, including CDR3 and Framework 4, which segments are added to the V-segment as a consequence of rearrangement of the heavy chain and light chain V-region genes during B-cell differentiation.

The sequences of the framework regions of different light or heavy chains are relatively conserved within a species. The framework region of an antibody, that is the combined framework regions of the constituent light and heavy chains, serves to position and align the CDRs in three dimensional space.

The amino acid sequences of the CDRs and framework regions can be determined using various well known definitions in the art, *e.g.,* Kabat, Chothia, international ImMunoGeneTics database (IMGT), and AbM (*see, e.g.,* Johnson *et al*., *supra;* Chothia & Lesk, 1987, Canonical structures for the hypervariable regions of immunoglobulins. J. Mol. Biol. 196, 901-917; Chothia C. et al., 1989, Conformations of immunoglobulin hypervariable regions. Nature 342, 877-883; Chothia C. et al., 1992, structural repertoire of the human VH segments J. Mol. Biol. 227, 799-817; Al-Lazikani et al., J.Mol.Biol 1997, 273(4)). Definitions of antigen combining sites are also described in the following: Ruiz et al., IMGT, the international ImMunoGeneTics database. Nucleic Acids Res., 28, 219-221 (2000); and Lefranc, M.-P. IMGT, the international ImMunoGeneTics database. Nucleic Acids Res. Jan 1;29(1):207-9 (2001); MacCallum et al, Antibody-antigen interactions: Contact analysis and binding site topography, J. Mol. Biol., 262 (5), 732-745 (1996); and Martin et al, Proc. Natl Acad. Sci. USA, 86, 9268-9272 (1989); Martin, et al, Methods Enzymol., 203, 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. *See, e.g*., Epitope Mapping Protocols in Methods in Molecular Biology, Vol. 66, Glenn E. Morris, Ed (1996).

As used herein, "chimeric antibody" refers to an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e.g.,* an enzyme, toxin, hormone, growth factor, drug, etc.; or (b) the variable region, or a portion thereof, is altered, replaced or exchanged with a variable region, or portion thereof, having a different or altered antigen specificity; or with corresponding sequences from another species or from another antibody class or subclass.

The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics such as a mixture of cells or a cell lysate. Thus, under designated immunoassay conditions, the specified antibodies bind to a particular protein (*e.g.*, SEQ ID NO:2) at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, Using Antibodies, A Laboratory Manual (1998) for a description of immunoassay formats and conditions that can be used to determine specific immunoreactivity).

"Antigen presenting cells", or "APCs" are cells that cells that mediate the cellular immune response by processing and presenting antigens to the T-cell receptor and include Langerhans cells, veiled cells of afferent lymphatics, dendritic cells and interdigitating cells of lymphoid organs. APCs include mononuclear cells such as lymphocytes and macrophages.

A polynucleotide or polypeptide sequence is "heterologous" to an organism or a second polynucleotide sequence if it originates from a foreign species, or, if from the same species, is modified from its original form. For example, when a heterologous promoter is said to be operably linked to a coding sequence, it means that the coding sequence is derived from one species whereas the promoter sequence is derived from another, different species; or, if both are derived from the same species, the coding sequence is not naturally associated with the promoter (*e.g.,* the promoter is a genetically engineered promoter or promoter fragment not found naturally associated with the coding sequence).

### BRIEF DESCRIPTION OF THE DRAWINGS

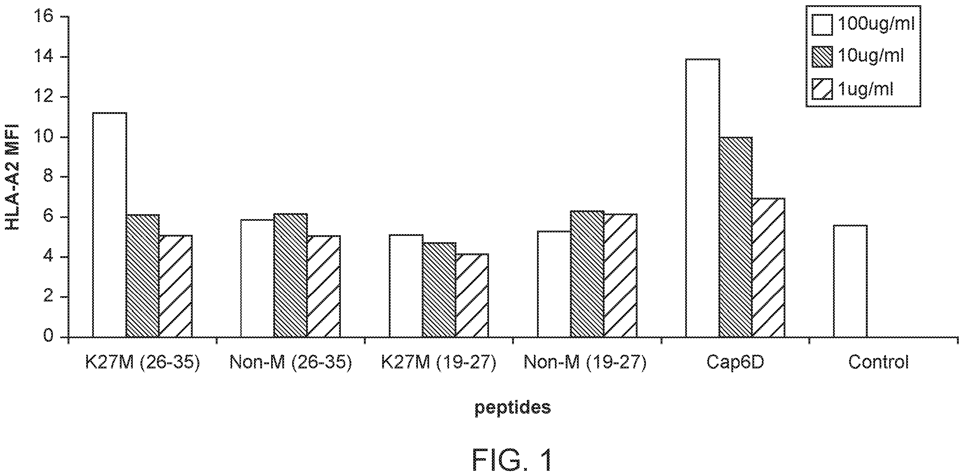

Figure 1. Identification of a HLA-A*0201-restricted epitope in H3.3 with the K27M mutation. A. HLA-A201 binding ability of H3.3-derived peptides was analyzed by T2 cell A2-binding assay. Cap1-6D is an altered peptide ligand, which has been derived from an epitope in human carcinoembryonic Ag, CEA605-613 and was used as positive control. B. H3.3 tetramer staining analysis of the CD8+ CTLs generated with the H3.3.K27M (26-35) after 1st antigen stimulation (left) and weekly re-stimulations (right).

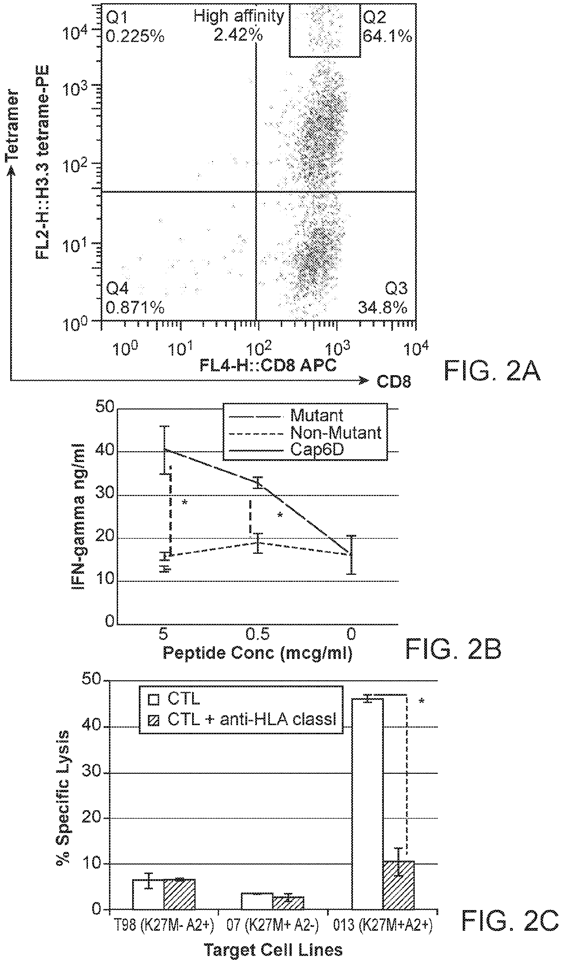

Figures 2A-C. HLA-A*0201+ donor-derived CTLs specifically recognize HLA-A*0201+ K27M+ glioma cells in an HLA-class I-dependent manner. Peripheral blood mononuclear cells from an HLA-A*0201+ donor were stimulated in vitro with the H3.3.K27M peptide and evaluated for their reactivity against: (1A) HLA-A*0201/H3.3.K27M-specific tetramer and anti-CD8 mAb, and (1B) T2 cells pulsed with the mutant or non-mutated H3.3 peptide by IFN-γ ELISA. In (2A), among the CD8+tetramer+ population (64.1% of total lymphocyte-gated cells), there is a tetramer^{high} subpopulation (2.4% of total lymphocyte-gated cells), some of which were used as CTL clones. In (2B), the Cap1-6D peptide (tested at 5 µg/ml only) is a high avidity HLA-A*0201-binding epitope derived from CEA4 used as an irrelevant negative control. (1C) The CTL line was evaluated for cytotoxicity against glioma cell lines T98 (HLA-A*0201+ but K27M-negative), HSJD-DIPG-07 (HLA-A*0201-negative but K27M+), and HSJD-DIPG-13 (HLA-A*0201+ and K27M+) lines. CFSE-labeled target cells (10e4/well) were incubated with CTLs at the E/T ratio of 25 for 4 hours. To block the CTL cytotoxicity, anti-HLA-ABC 10µg/ml was added to one group. At the end of incubation, 7-ADD was added into each well and incubated for 10 minutes on ice. The samples were analyzed by flow cytometry, and the killed target cells were identified as CFSE+ and 7-ADD+ cells. The cytotoxicity was calculated as the percentage of CFSE+ and 7-ADD+ cells in total HLA-A*0201+ CFSE+ cells. (*p<0.05 by Wilcoxon rank-sum tests).

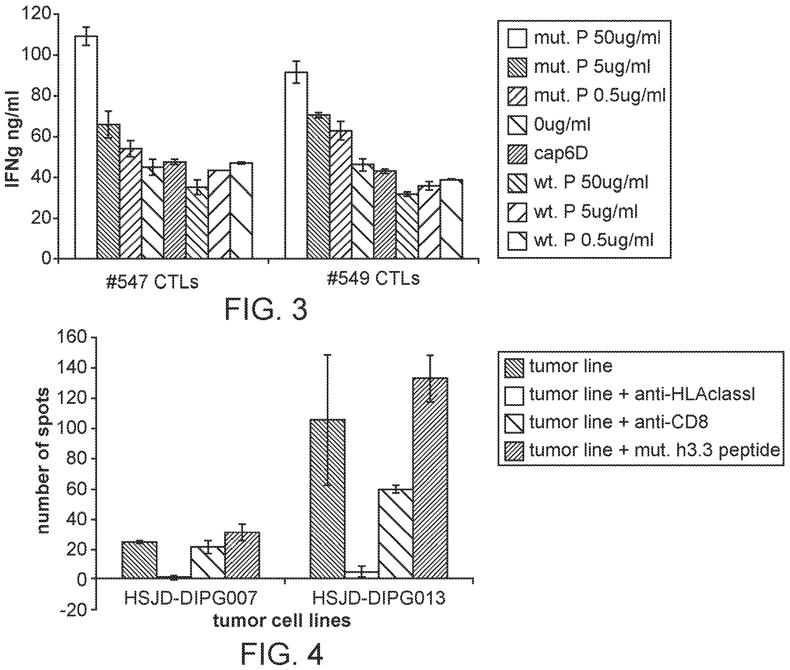

Figure 3. CD8+ T cell lines stimulated with the H3.3.K27M (26-35) peptide recognize the H3.3.K27M (26-35) but not the non-mutant counterpart. CD8+ T cell lines were induced and expanded with the H3.3.K27M (26-35) peptide from the PBMCs derived from two HLA-A*0201+ healthy donors (#547 and #549). T2 cells pulsed with the mutant H3.3.K27M (26-35) peptide or the non-mutant H3.3.non-M (26-35) peptide at the indicated concentrations were mixed with the CD8+ T cell lines for 24 hours. IFN-γ in the supernatants were assessed by ELISA.
Figure 4. HLA-A2+ donor-derived CTLs specifically recognize HLA-A2+ K27M+ glioma cells and secrete IFN-γ in an HLA-A2- and CD8- dependent manners. Peripheral blood mononuclear cells from an HLA-A2 donor were stimulated in vitro with synthetic peptides for H3.3.K27M (26-35) and evaluated their reactivity against HSID-007 (HLA-A2-negative but K27M+) or HSID-013 (HLA-A2+ and K27M+) lines by IFN-γ ELISPOT assays. Anti-HLA-ABC (10µg/ml) and anti-CD8 antibodies were also used to evaluate whether the response is dependent on HLA-A2 and CD8+, respectively.

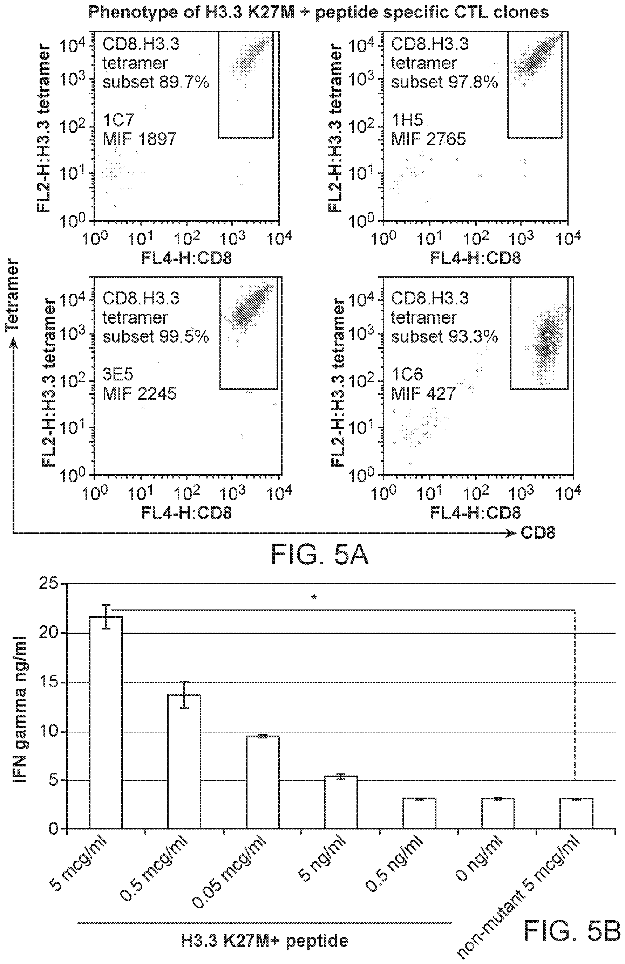

Figure 5A-B Characterization of H3.3.K27M-specific CTL clones. (5A) Clones were generated by limiting dilution cloning of HLA-A2-H3.3.K27M-tetramer-positive single cells from H3.3 K27M-specific CTLs using FACS-sorting. Clones with relatively high (1C7, IH5 and 3E5) and moderate (1C6) affinity based on the mean fluorescence index (MFI) were selected for further evaluations. (5B) An H3.3.K27M-specific CTL clone (IH5) demonstrates H3.3.K27M-specific reactivity as shown by IFN-γ ELISA against T2 cells pulsed with the mutant H3.3 K27M+ peptide at titrating concentrations and the wild-type peptide (H3.3 K27M-negative; used at 500 ng/ml).

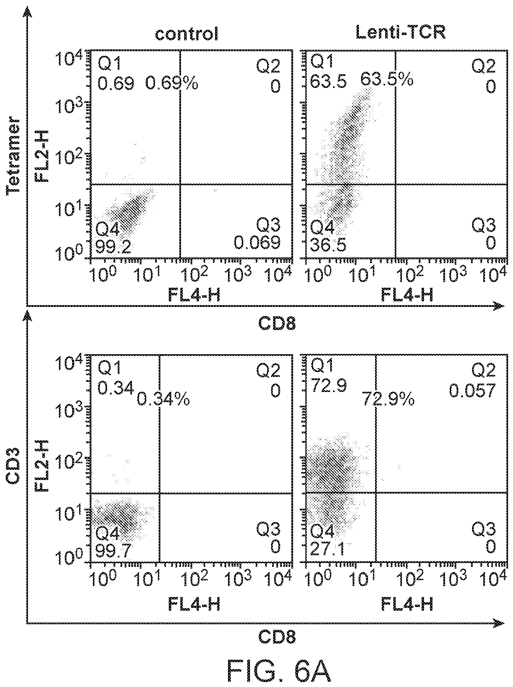

Figure 6A-C. Evaluation of H3.3.K27M-specific TCR. (6A), J.RT-T3.5 cells were transduced with lentiviral vector encoding the TCR α- or β-chains derived from H3.3.K27M-specific CTL clone IH5 (J.RT-T3.5-TCR). The J.RT-T3.5-TCR or control non-transduced J.RT-T3.5 cells were evaluated for the surface TCR expression using PE-labeled HLA-A*0201/ H3.3.K27M tetramer (upper panel) or PE-labeled anti-CD3 mAb (lower panel) and FITC-labeled anti-human CD8 mAb (upper and lower panels). Since J.RT3-T3.5 cells are CD4+ and CD8-negative, tetramer+ CD8-negative cells are ones expressing the transgene-derived TCR. CD3-upregulation indicates activation of cells. (6B), J.RT-T3.5-TCR, but not control J.RT-T35 cells, upregulate CD69 expression upon recognition of the H3.3 K27M peptide loaded on T2 cells. (3C), DIPG 13 cells [HLA-A*0201+ (albeit dim), K27M mutation+] were incubated with J.RT-T3.5-TCR or control J.RT-T3.5 cells. IL-2 secretion in the culture media was assayed by specific ELISA.

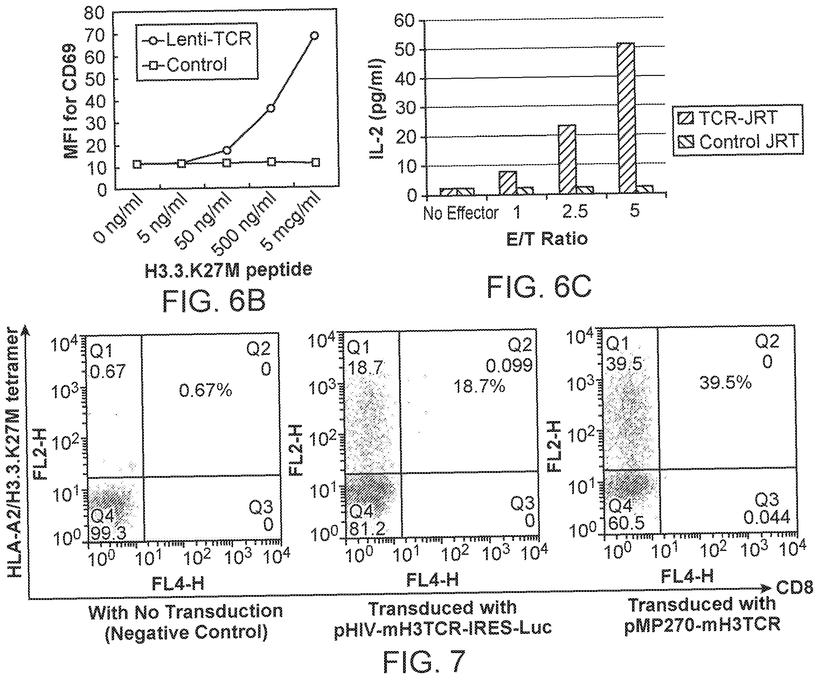

Figure 7. Expression of transgene-derived TCR. J.RT3-T3.5 cells, which are deficient for endogenous TCR β-chain, were transduced with lentiviral vectors (pHIV-mH3TCR-IRES-Luc or pMP270-mH3TCR) encoding TCR α- and β-chains derived from an H3.3.K27M-specific CTL clone (IH5; Figure 7) and evaluated for the surface TCR expression using PE-labeled HLA-A2/ H3.3.K27M tetramer and FITC-labeled anti-human CD8 mAb. Since J.RT3-T3.5 cells are CD4+ and CD8-negative, tetramer+ CD8-negative cells are ones expressing the transgene-derived TCR. Negative control cells are non-transfected cells stained with the same tetramer indicting the specificity of the tetramer-binding.

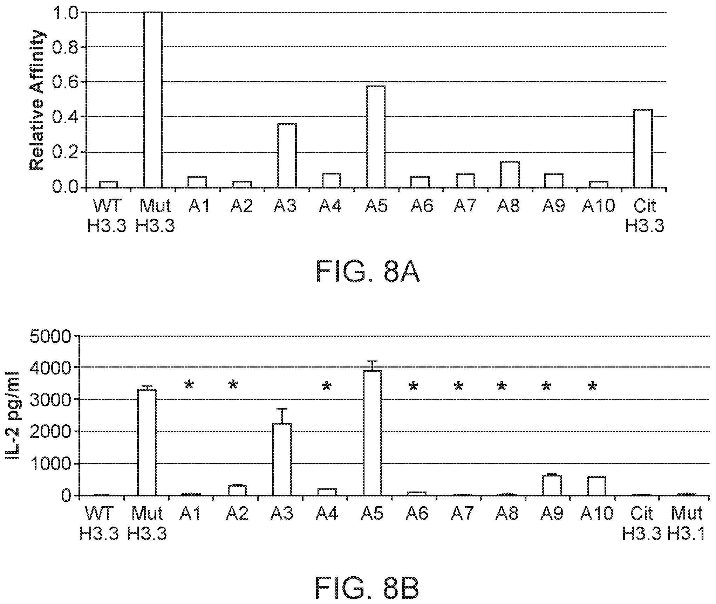

Figure 8A-B. Alanine scanning to determine the key immunogenic AA residues of the H3.3.K27M epitope. (8A) Relative HLA-A2-binding affinity of each peptide to that of H3.3.K27M (26-35) was determined by cell-free binding assay using HLA-A2 purified by affinity chromatography from the EBV transformed homozygous cell line JY. (8B), J.RT3-T3.5 cells were transduced with lentiviral vector encoding the H3.3.K27M-specific TCR and evaluated for the recognition of each peptide loaded on T2 cells by production of IL-2. Each group was assayed as triplicate * <0.05 by Student-t compared with the mutant H.3.3. In addition to 10 synthetic peptides each containing the substitution with alanine (A1-A10), we also evaluated synthetic peptides designed for citrullinated H3.3. K27M epitope (Cit H3.3; *i.e.,* the first AA of the H3.3.K27M epitope is replaced by citrulline) and H3.1 (a homologue of H3.3.) derived K27M epitope (Mut H.3.1).

### DETAILED DESCRIPTION OF THE INVENTION

### Introduction

The inventors have found that a peptide that encompasses amino-acid positions 26-35 of H3.3, which includes the K27M mutation [referred to herein as "H3.3.K27M (26-35)"], can induce specific cytotoxic T lymphocyte (CTL) responses in human leukocyte antigen (HLA)-A2+ donors. Furthermore, CTLs against H3.3.K27M (26-35) recognize HLA-A2+ glioma cell lines that also harbor the K27M mutation. Accordingly, described herein are compositions for use in generating an immune response in human subjects to a peptide comprising amino-acid positions of 26-35 of H3.3 or variants thereof.

### CTL Peptides

The CTL peptides described herein comprise (R/A)MSAP(S/A)TGGV (SEQ ID NO:1), where the amino acid options in parentheses are alternative options for the specified position. Accordingly, CTL peptides can comprise RMSAPSTGGV (SEQ ID NO:4), AMSAPSTGGV (SEQ ID NO:5), RMSAPATGGV (SEQ ID NO:6), or AMSAPATGGV (SEQ ID NO:7). CTL peptides comprising RMSAPSTGGV (SEQ ID NO:4) or AMSAPSTGGV (SEQ ID NO:5) represent peptides for targeting H3.3.K27M (26-35). CTL peptides comprising RMSAPATGGV (SEQ ID NO:6), or AMSAPATGGV (SEQ ID NO:7) represent peptides for targeting the corresponding H3.1 K27M (26-35). The length of the CTL peptides can vary so long as the peptides are effective at inducing an immune response, e.g., a CTL response. In some aspects, the peptide will consist of 100 or fewer or 50 or fewer amino acids. In some aspects, the CTL peptides will have 10-14 amino acids, i.e., will be 10, 11, 12, 13, or 14 amino acids long. As SEQ ID NO:1 is 10 amino acids long, the 11 or 14-amino acid options will have one to four additional amino acids on the amino or carboxyl terminus of SEQ ID NO:1, or in some aspects, one or two additional amino acid on each of the amino and carboxyl terminus of SEQ ID NO:1. Additional amino acids can be selected from any of the twenty naturally-occurring amino acids or can be a non-naturally-occurring amino acid.

The peptides can be modified to alter, for example, their in vivo stability. For instance, inclusion of one or more D-amino acids in the peptide typically increases stability, particularly if the D-amino acid residues are substituted at one or both termini of the peptide sequence. Stability can be assayed in a variety of ways such as by measuring the half-life of the proteins during incubation with peptidases or human plasma or serum. A number of such protein stability assays have been described (see, e.g., Verhoef et al., Eur. J. DrugMetab. Pharmacokin. 11:291-302 (1986).).

The peptides can also be modified by linkage to other molecules. For example, different N- or C-terminal groups may be introduced to alter the molecule's physical and/or chemical properties. Such alterations may be utilized to affect, for example, adhesion, stability, bioavailability, localization or detection of the molecules. For diagnostic purposes, a wide variety of labels may be linked to the terminus, which may provide, directly or indirectly, a detectable signal. Thus, the peptides of the subject invention may be modified in a variety of ways for a variety of end purposes while still retaining biological activity.

The following examples of chemical derivatives are provided by way of illustration and not by way of limitation.

Aromatic amino acids may be replaced with D- or L-naphylalanine, D- or L-Phenylglycine, D- or L-2-thieneyl-alanine, D- or L-1-, 2-, 3- or 4-pyreneylalanine, D- or L-3-thieneylalanine, D- or L- (2-pyridinyl)-alanine, D- or L- (3-pyridinyl)-alanine, D- or L- (2-pyrazinyl)-alanine, D- or L- (4-isopropyl)-phenylglycine, D-(trifluoromethyl)-phenyl-glycine, D-(trifluoromethyl)-phenylalanine, D-p-fluoro-phenylalanine, D- or L-p-biphenylphenylalanine, D- or L-p-methoxybiphenylphenylalanine, D- or L-2-indole-(alkyl)alanines, and D- or L-alkylamines where alkyl may be substituted or unsubstituted methyl, ethyl, propyl, hexyl, butyl, pentyl, iso-propyl, iso-butyl, sec-isotyl, iso-pentyl, non-acidic amino acids, of C1-C20.

Acidic amino acids can be substituted with non-carboxylate amino acids while maintaining a negative charge, and derivatives or analogs thereof, such as the non-limiting examples of (phosphono) -alanine, glycine, leucine, isoleucine, threonine, or serine; or sulfated (e.g., -SO₃H) threonine, serine, tyrosine.

Other substitutions may include unnatural hyroxylated amino acids made by combining "alkyl" (as defined and exemplified herein) with any natural amino acid. Basic amino acids may be substituted with alkyl groups at any position of the naturally occurring amino acids lysine, arginine, ornithine, citrulline, or (guanidino) -acetic acid, or other (guanidino) alkyl-acetic acids, where "alkyl" is define as above. Nitrile derivatives (e.g., containing the CN-moiety in place of COOH) may also be substituted for asparagine or glutamine, and methionine sulfoxide may be substituted for methionine. Methods of preparation of such peptide derivatives are well known to one skilled in the art.

In addition, any amide linkage can be replaced by a ketomethylene moiety, *e.g.,* (-C(=O) -CH₂-) for (- (C=O) -NH-) . Such derivatives are expected to have the property of increased stability to degradation by enzymes, and therefore possess advantages for the formulation of compounds which may have increased in vivo half-lives, as administered by oral, intravenous, intramuscular, intraperitoneal, topical, rectal, intraocular, or other routes.

In addition, any amino acid can be replaced by the same amino acid but of the opposite chirality. Thus, any amino acid naturally occurring in the L-configuration (which may also be referred to as the R or S configuration, depending upon the structure of the chemical entity) may be replaced with an amino acid of the same chemical structural type, but of the opposite chirality, generally referred to as the D- amino acid but which can additionally be referred to as the R- or the S-, depending upon its composition and chemical configuration. Such derivatives have the property of greatly increased stability to degradation by enzymes, and therefore are advantageous in the formulation of compounds which may have longer in vivo half-lives, when administered by oral, intravenous, intramuscular, intraperitoneal, topical, rectal, intraocular, or other routes.

Fusion peptides including an antigenic peptide as described above fused to another peptide sequence are specifically contemplated for use with the methods and compositions described herein. In some aspects, peptides include fusion peptides composed of a CTL sequence as described herein (e.g., SEQ ID NO:1) and a helper T lymphocyte (CD4) epitope sequence fused together. Examples of suitable CD4 epitopes include the synthetic sequence PADRE, tetanus-specific peptides, peptides derived from the same antigen or other antigens from the virus that is to be targeted. Similarly, for cancer antigens, a CD4 peptide derived from the same antigen, or any other cell-antigen known in the art, and the like may be used. Linker peptide sequences at the N- or C-terminal end of the fusion or between the CTL and CD4 epitopes in the fusion also may be used. Such linker sequences generally can be, for example, from about 1 to about 10 amino acids in length and, e.g., about 2 to about 7 amino acids or from about 3 to about 5 amino acids in length can optionally comprise modified or non-traditional amino acids.

Also described are peptide/HLA-A2 multimers, and in some aspects, their use to isolate peptide-specific CTLs. "Multimers" include, e.g., tetramers, pentamers and any number of MHC-peptide structure assembled around the core molecule with fluorochrome. In some aspects, the selected MHC molecules (e.g. HLA-A2) along with beta2-microgloblin are assembled around one core molecule which connects a fluorochrome molecule (such as FITC, so that the multimer will fluoresce) and multiple MHC-beta-microgloblin molecules (in case of tetramer and pentamer, there will be 4 and 5, respectively). Then, the MHC molecule is also bound with the peptide so that the MHC-peptide complex on the multimer molecule can be recognized by the TCR on T-cells. See, *e.g.,* Wooldridge, et al., Immunology 126(2): 147-164 (2009); Yokouchi, et al., Cancer Sci. 97(2): 148-54 (2006). Accordingly, in some aspects, fluorochrome-conjugated peptide-major histocompatibility complex (pMHC) multimers, wherein the peptide is a CTL peptide (e.g., comprising SEQ ID NO:1) are described.

In some aspects, T-cell populations are enriched for T-cells expressing one or more TCRs that bind to the CTL peptide/MHC complex. For example, in some aspects, a T-cell population is cultured in the presence of the CTL peptide (either the naked peptide or peptide loaded onto APCs), thereby preferentially stimulating division of T-cells carrying TCRs that bind the peptide/MHC complex. In some aspects, the culturing occurs in the presence of IL-2, IL-4, IL-7 or IL-15 alone, or of 2-way, 3-way, or 4-way combinations thereof. T-cell populations expanded in this way will be enriched for TCRs that bind the protein/MHC complex. Subsequently, fluorochrome-conjugated peptide-major histocompatibility complex (pMHC) multimers can be used to label the T-cells expressing the TCRs that bind the peptide/MHC complex, and can be sorted, for example by FACS.

Exemplary TCR alpha and beta chain sequences that recognize the H3.3.K27M epitope are provided below with CDR1, CDR2, and CDR3 underlined.
**TCRA-Va19*01/J43**
CDR1: T R D T T Y Y (SEQ ID NO:12);
CDR2: R N S F (SEQ ID NO:13)
CDR3: A L S E (SEQ ID NO:14)
**Coding sequence of the above amino acid sequence:**
**TCRB-Vb27/J2.7**
CDR1: N Met N H E Y (SEQ ID NO:15);
CDR2: Y S Met N V E V T (SEQ ID NO: 16)
CDR3: C A S G W G G P F Y E Q Y (SEQ ID NO:17)
**Coding sequence of the above amino acid sequence:**

### Polynucleotides

Polynucleotides encoding the CTL peptides described herein are also described and are referred to as "CTL polynucleotides". In some aspects, the CTL polynucleotides can be a DNA or RNA sequence. The CTL polynucleotide is can be operably linked to some or all of transcriptional and translational regulatory elements, such as a promoter, enhancer and polyadenylation sequence. Regulatory sequences are art-recognized and are described, e.g., in Goeddel; Gene Expression Technology: Methods in Enzymology 185, Academic Press, San Diego, CA (1990). In some aspects, the promoter is a constitutive promoter, e.g., a strong viral promoter, e.g., CMV promoter. The promoter can also be cell- or tissue-specific, that permits substantial transcription of the DNA only in predetermined cells, e.g., in antigen presenting cells, such as the dendritic cell-specific CD1 lc promoter described in Brocker T, J. Leuk. Biology 66:331 -335, 1999. The promoter can also be an inducible promoter, for example, a metallothionein promoter. Other inducible promoters include those that are controlled by the inducible binding, or activation, of a transcription factor, e.g., as described in U.S. Patent Nos. 5,869,337 and 5,830,462 by Crabtree et al., describing small molecule inducible gene expression (a genetic switch); International patent applications PCT/US94/01617, PCT/US95/10591, PCT/US96/09948 and the like, as well as in other heterologous transcription systems such as those involving tetracyclin-based regulation reported by Bujard et al., generally referred to as an allosteric "off-switch" described by Gossen and Bujard, Proc. Natl. Acad. Sci. U.S.A. (1992) 89:5547 and in U.S. Patents 5,464,758; 5,650,298; and 5,589,362 by Bujard et al. Other inducible transcription systems involve steroid or other hormone-based regulation.

The CTL polynucleotides may also be produced in part or in total by chemical synthesis, e.g., by the phosphoramidite method described by Beaucage and Carruthers, Tetra. Letts., 22:1859-1862 (1981) or the triester method according to the method described by Matteucci et al., J. Am. Chem. Soc, 103:3185 (1981), and may be performed on commercial automated oligonucleotide synthesizers. A double-stranded fragment may be obtained from the single stranded product of chemical synthesis either by synthesizing the complementary strand and annealing the strand together under appropriate conditions or by adding the complementary strand using DNA polymerase with an appropriate primer sequence.

The CTL polynucleotide operably linked to all necessary transcriptional and translational regulation elements can be injected as naked DNA into a subject or contacted *in vitro* with antigen presenting cells. In some aspects, the CTL polynucleotide and regulatory elements are present in a plasmid or vector. Thus, the CTL polynucleotide can be DNA, which is itself non-replicating, but is inserted into a plasmid, which may further comprise a replicator. The DNA can be a sequence engineered so as not to integrate into the host cell genome. Exemplary vectors are expression vectors, *i.e.,* vectors that allow expression of a nucleic acid in a cell. Exemplary expression vectors are those which contain both prokaryotic sequences, to facilitate the propagation of the vector in bacteria, and one or more eukaryotic transcription units that are expressed in eukaryotic cells.

Alternatively, derivatives of viruses such as the bovine papillomaviras (BPV-1), of Epstein- Barr virus (pHEBo, pREP-derived and p205) can be used for transient expression of proteins in eukaryotic cells. These viruses expressing the CTL polypeptides can be used to infect APCs, which are then administered to patients. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press: 1989), Chapters 16 and 17.

In some aspects, the CTL polynucleotide is expressed in a prokaryote. In some aspects, the polypeptide is expressed in *E. coli.* In some aspects, the CTL polypeptide is expressed in *Listeria monocytogenes,* which in some aspects, is attenuated, and which can be administered to a human individual to provide the CTL peptide to the individual. *See, e.g.,* US Patent Publication No. US2013/0259891. In other aspects, the CTL polynucleotide is expressed in a eukaryotic cell. For example, the CTL polynucleotide and polypeptide can be expressed in yeast, insect cells, animal cells, including mammalian cells, *e.g.,* human cells.

### Inducing an immune response

The CTL peptides of the invention are capable of inducing an immune response when administered to an animal (e.g., a human). An exemplary immune response is a CTL response. The CTL peptides, once introduced can be processed and presented to the MHC class I complex of an antigen presenting cell. Human MHC class I complex includes HLA-A, B, and C alleles. The CTL peptides (*e.g.,* SEQ ID NO:1) have higher affinity to HLA-A2+ individuals. See Table 1. The MHC class I-bound epitope is then transported to the cell surface and recognized by cytotoxic T lymphocytes (CTLs) through T cell receptors (TCRs) located on their surface. Recognition of an antigen/MHC complex by the TCR triggers a cascade of protein and cytokine interactions leading to, among other interactions, the activation, maturation and proliferation of the precursor CTLs, resulting in CTL clones capable of destroying the cells exhibiting CTL peptides recognized as foreign.

In one aspect, one or more CTL peptides as described herein are administered to the patient. In another aspect, one or more polynucleotides encoding one or more CTLs are introduced to APCs, and these APCs expressing CTLs are administered into a human patient to induce an immune response, the cytotoxic T cell response. In some aspects, the CTL peptides are loaded onto the APCs without expressing the CTL peptides in the cell. *See, e.g.,* US Patent No. 8,652,462. In some aspects, the APCs are harvested from an individual, loaded with the CTL peptides (or the CTL polynucleotides are introduced into the APCs), and then the APCs are introduced back into the individual (*i.e.,* the cells are autologous) and a CTL immune response is induced in the individual to the CTL polypeptide.

In one aspect, an immune response is induced by directly administering the CTL peptides to patients. Adjuvants and/or nonspecific inflammatory mediators are not be required, but can be optionally administered with the active ingredient before, during, or after the priming and/or boosting of the immune response. Adjuvants are substances that are used to specifically or nonspecifically potentiate an antigen-specific immune response, perhaps through activation of antigen presenting cells. The adjuvant can be, e.g., Montanide-ISA 51 (e.g., from Seppic Hiltonol (e.g., from Oncovir), anti-CD40 agonistic monoclonal antibodies, polyICLC, Bacillus Calmette-Guerin (BCG) vaccine, an ADP-ribosylating exotoxin (e.g., cholera toxin, diphtheria toxin, E. coli heat-labile enterotoxin, pertussis toxin, *P. aeruginosa* exotoxin A), a fragment thereof containing the A and/or B subunit, a chemically modified or genetically mutated derivative thereof, or a derivative thereof with reduced toxicity; a chemical conjugate or genetic recombinant containing a bacterial ADP-ribosylating exotoxin or derivative thereof; a chemokine (e.g., defensins, HCC-1, HCC-4, MCP-1 MCP-3, MCP-4, MLP-1α, MIP-1β, MIP-1γ, MIP-3α, MIP-2, RANTES); another ligand of a chemokine receptor (e.g., CCR1, CCR-2, CCR-5, CCR-6, CXCR-1); a cytokine (e.g., IL-1β, IL -2, ÏL-6, IL-8, IL-10, IL-12; IFN-γ; TNF-α; GM-CSF); another ligand of a cytokine receptor; a salt (e.g., aluminum hydroxide or phosphate, calcium phosphate); lipid A or a derivative thereof (e.g., monophosphoryl or diphosphoryl lipid A, lipid A analogs, AGP, ASO2, ASO4, DC-Choi, Detox, OM-174); a pathogen-associated molecular pattern (PAMP); immunostimulatory CpG motifs in bacterial DNA or an oligonucleotide (see, for example, U.S. Patent 6,218,371); a Leishmania homolog of elF4a or a derivative thereof (see, for example, U.S. Patent 5,876,735); a heat shock protein or derivative thereof; C3d tandem array; a muramyl dipeptide (MDP) or a derivative thereof (e.g., murabutide, threonyl-MDP, muramyl tripeptide); ISCOMS and saponins (e.g., Quil A, QS-21); squalene; superantigens; a ligand of a toll-like receptor, or the like. Adjuvants may be chosen to preferentially induce antibody or cellular effectors, specific antibody isotypes (e.g., IgM, IgD, IgAl, IgA2, secretory IgA, IgE, IgG1, IgG2, IgG3, and/or IgG4), or specific T-cell subsets (e.g., CTL, Thl, Th2 and/or TDTH)- For example, antigen presenting cells may present Class I1-restricted antigen to precursor CD4+ T cells, and the Th1 or Th2 pathway may be entered.

In another aspect, described herein is a method of administering APC cells expressing and/or presenting CTL peptides to induce immune response. Methods for delivering CTL polynucleotide to APCs are well-known in the art, for example, retroviruses, adenoviruses, lentiviruses, adeno-associated virus (AAV), and herpes simplex virus- 1, vaccinia viruses, and canarypox or fowlpox viruses can infect APC. Most of these vectors cause transient gene expression lasting less than two weeks (Bonnet et al, 2000). To initiate an immune response, expression of peptides, proteins, or MHC molecules may only need to last about three to ten days (*see e.g.,* U.S. Patents 5,656,465 and 5,833,975). Plasmids may also be used to transfer a gene into the cell by itself or with chemicals that enhance transfection, or via carriers. For example, cationic lipids, calcium phosphate, DEAE- dextran, polybrene-DMSO, or polycation amino acids (e.g., polylysine) are chemical transfectants.

The method may further involve a targeting molecule that preferentially binds to a target cell, for example antigen presenting cells. Such targeting mechanisms may include terminal galactosyl residues binding to asialoglycoprotein receptor (e.g., galactosylated nucleic acid and/or antigen), high-mannose oligosaccharide binding to mannose receptor (e.g., mannosylated nucleic acid and/or antigen), ligand binding to an Fc receptor (e.g., nucleic acid and/or antigen fused or linked to IgG constant region or other ligand of CD64), or membrane proteins highly expressed on antigen presenting cells (e.g., nucleic acid and/or antigen fused or linked to ligand or antibody specific for the membrane protein). Mannose receptors are exemplary targets because they are highly expressed on dendritic cells (especially Langerhans cells), and involved in antigen uptake. This significantly increases the APC's ability to capture exogenous proteins and process them (Sallusto, 1995). Antigen may be delivered to phagocytic cells of the skin such as, for example, Langerhans cells, other dendritic cells, macrophages, and other antigen presenting cells in the epidermis and dermis; antigen may also be delivered to phagocytic cells of the liver, spleen, and bone marrow that are known to serve as the antigen presenting cells through the blood stream or lymphatic system.

In a further aspect, the composition can comprise the CTL polypeptide and also comprise the universal T cell epitope called PADRE® (Epimmune, San Diego; described, for example in US Patent No. 5,736,142 or International Application WO95/07707. A "PanDR binding peptide" or "PADRE® peptide" is a member of a family of molecules that binds more than one HLA class II DR molecule. The pattern that defines the PADRE® family of molecules can be thought of as an HLA Class U supermotif. PADRE® binds to most HLA-DR molecules and stimulates in vitro and in vivo human helper T lymphocyte (HTL) responses. Alternatively T-helper epitopes can be used from universally used vaccines such as tetanus toxoid.

Typically, a vaccine or vaccine composition is prepared as an injectable, either as a liquid solution or suspension. Injection may be subcutaneous, intramuscular, intravenous, intraperitoneal, intrathecal, intradermal, intraepidermal, or by "gene gun". Other types of administration comprise electroporation, implantation, suppositories, oral ingestion, enteric application, inhalation, aerosolization or nasal spray or drops. Solid forms, suitable for dissolving in, or suspension in, liquid vehicles prior to injection may also be prepared. The preparation may also be emulsified or encapsulated in liposomes for enhancing adjuvant effect.

A liquid formulation may include oils, polymers, vitamins, carbohydrates, amino acids, salts, buffers, albumin, surfactants, or bulking agents. Exemplary carbohydrates include sugar or sugar alcohols such as mono-, di-, or polysaccharides, or water-soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethyl cellulose, or mixtures thereof. "Sugar alcohol" is defined as a C4 to C8 hydrocarbon having an -OH group and includes galactitol, inositol, mannitol, xylitol, sorbitol, glycerol, and arabitol. These sugars or sugar alcohols mentioned above may be used individually or in combination. There is no fixed limit to the amount used as long as the sugar or sugar alcohol is soluble in the aqueous preparation. In some aspects, the sugar or sugar alcohol concentration is between 1,0 % (w/v) and 7,0 % (w/v), e.g., between 2,0 and 6,0 % (w/v). Exemplary amino acids include levorotary (L) forms of carnitine, arginine, and betaine; however, other amino acids may be added. Exemplary polymers include polyvinylpyrrolidone (PVP) with an average molecular weight between 2,000 and 3,000, or polyethylene glycol (PEG) with an average molecular weight between 3,000 and 5,000. In some aspects, one can use a buffer in the composition to minimize pH changes in the solution before lyophilization or after reconstitution. Any physiological buffer may be used, but in some cases can be selected form citrate, phosphate, succinate, and glutamate buffers or mixtures thereof. Surfactants that can be added to the formulation are shown in EP patent applications No. EP 0 270 799 and EP 0 268 110.

Additionally, polypeptides can be chemically modified by covalent conjugation to a polymer to increase their circulating half-life, for example. Exemplary polymers, and methods to attach them to peptides, are shown in U.S. Patent Nos.4,766,106; 4,179,337; 4,495,285; and 4,609,546. Exemplary polymers are polyoxyethylated polyols and polyethylene glycol (PEG). PEG is soluble in water at room temperature and has the general formula: R(O-CH₂-CH₂)ₙO-R where R can be hydrogen, or a protective group such as an alkyl or alkanol group. In some aspects, the protective group has between 1 and 8 carbons, e.g., methyl. The symbol n is a positive integer, preferably between 1 and 1000, e.g., between 2 and 500. The PEG can have, for example, average molecular weight between 1000 and 40,000, *e.g.,* between 2000 and 20,000, *e.g.,* between 3,000 and 12,000. In some aspects, PEG has at least one hydroxyl group, *e.g.,* it has a terminal hydroxy group.

Water soluble polyoxyethylated polyols are also useful and can be linked to the CTL polypeptides described herein. They include polyoxyethylated sorbitol, polyoxyethylated glucose, polyoxyethylated glycerol (POG), etc. Another drug delivery system for increasing circulatory half-life is the liposome. The peptides and nucleic acids of the invention may also be administered via liposomes, which serve to target a particular tissue, such as lymphoid tissue, or to target selectively infected cells, as well as to increase the half-life of the peptide and nucleic acids composition. Liposomes include emulsions, foams, micelles, insoluble monolayers, liquid crystals, phospholipid dispersions, lamellar layers and the like. Liposomes either filled or decorated with a desired peptide or nucleic acids of the invention can be directed to the site of lymphoid cells, where the liposomes then deliver the peptide and nucleic acids compositions. Liposomes for use in accordance with the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., 1980, and U.S. Patent Nos. 4,235,871, 4,501,728, 4,837,028, and 5,019,369.

For targeting cells of the immune system, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

After the liquid pharmaceutical composition is prepared, the composition can be lyophilized to prevent degradation and to preserve sterility. Just prior to use, the composition may be reconstituted with a sterile diluent (Ringer's solution, distilled water, or sterile saline, for example) which may include additional ingredients. Upon reconstitution, the composition can be administered to subjects.

The polypeptide and APCs of the invention can be used to treat individuals having gliomas. In some embodiments, the gliomas are selected from glioblastoma (GBM) and diffuse intrinsic pontine gliomas (DIPG). In some aspects, the individual is a HLA-A2+ type. The HLA genes are the human versions of the major histocompatibility complex (MHC) genes that are found in most vertebrates (and thus are the most studied of the MHC genes). HLAs corresponding to MHC class I are HLA-A, HLA-B, and HLA-C. HLAs corresponding to MHC class II are DP, DM, DOA, DOB, DQ, and DR. Tests for HLA typing are readily available and can be used to screen patients who are likely benefit from the treatment, for example, from http://labtestsonline.org/understanding/analytes/hla-testing/tab/test/. In some cases, the patient may also have clinical symptoms besides gliomas. The glioma patient may belong to any age group, including children (e.g., 0-18 years old).

### Antibodies recognizing the CTL peptides

Also described is an antibody or an antigen-binding fragment thereof, that recognizes the CTL peptides described herein. Antigen-binding fragments of antibodies encompass fragments which comprise the hypervariable domains designated CDRs (Complementarity Determining Regions) or part(s) thereof encompassing the recognition site for the antigen, i.e., the CTL peptides described herein, thereby defining antigen recognition specificity. Each Light and Heavy chain (respectively VL and VH) of a four-chain immunoglobulin has three CDRs, designated VL-CDR1 , VL-CDR2, VL- CDR3 and VH-CDR1 , VH-CDR2, VH-CDR3, respectively. Thus, in some cases the antibody comprises fragments of antibodies described herein (antigen-binding fragments), which comprise or consist of all or a selection of CDRs among VL-CDR1 , VL-CDR2, VL-CDR3 and VH-CDR1 , VH-CDR2 and VH-CDR3 or functional portions thereof, *i.e.* portions that exhibit the desired binding specificity, preferably with a high affinity, for the CTL peptides of the invention. For example, in some aspects, the antibody or antibody fragment binds to SEQ ID NO:2 but does not bind to (*e.g.,* binds at no higher than background) SEQ ID NO:3.

The antibody may be produced by any well-known method for antibody production. For example, the antibody may be obtained by administration of any of the above aspects of CTL peptides into an animal and harvesting the antibodies produced as a result.

### Examples

### Example 1. The H3.3.K27M-derived HLA-A*0201-restricted cytotoxic T-lymphocyte (CTL) epitope and cloning of T-cell receptor (TCR) cDNAs

### 1. Screening of HLA-A*0201-binding epitopes in H3.3 with the K27M mutation

Using the NetMHC 3.4 server (http://www.cbs.dtu.dk/services/NetMHC/), an artificial neural network-based prediction system of peptide binding motifs to HLAs, we predicted that an H3.3-derived 10-mer amino acid (AA) peptide which encompasses the AA position 26-35 including the K27M mutation [H3.3.K27M (26-35)] will serve as a potent epitope in the context of HLA-A*0201, while the non-mutant counterpart for the corresponding positions [H3.3.Non-M (26-35)] was not predicted to have high affinity to HLA-A*0201 (Table 1).

| **Table 1.** The H3.3 (26-35) peptide Binding Scores | | logscore | affinity(nM) |
|---|---|---|---|
| K27M Mutant: | RMSAPSTGGV | 0.477 | 285 |
| Non-mutant: | RKSAPSTGGV | 0.073 | 22651 |

Analysis of H3.3.K27M (26-35), H3.3.Non-M (26-35) peptides as well as 9-mer peptides H3.3.K27M (19-27) and H3.3.Non-M (19-27). We next directly evaluated relative binding affinity of these peptides using the transporter associated with antigen processing (TAP)-deficient HLA-A*0201+ T2 cell line. Since stable binding of HLA-A*0201 with peptide epitopes further stabilizes the surface expression of HLA-A*0201, quantitative expression levels of HLA-A*0201 correlate with the binding affinity of the peptide-epitopes that are co-incubated with the T2 cells (**Figure 1**). The MFI values for T2 cells with no peptide indicate the baseline HLA-A2 expression level. Cap6D is a well-documented HLA-A*0201-binding epitope and used as a positive control for the assay. The H3.3.K27M (26-35), but none of other H3.3-derived peptides, demonstrated a peptide-dose-dependent increase of mean fluorescence intensity (MFI). These data suggested that H3.3.K27M (26-35) peptide is a potential epitope for specific T-cell responses. To precisely measure the binding of peptides to HLA-A*02:01 class I molecules, we performed a competitive binding inhibition assay, and evaluated the concentration of peptide yielding 50% inhibition of the binding of the radiolabeled probe peptide (IC50). The mutant H3.3.K27M peptide constantly demonstrated IC50 of 95nM-187nM in 4 separate samples, which are considered to be excellent binding capabilities. On the other hand, the non-mutant H3.3 peptide (on the corresponding position) demonstrated >30 fold lower binding capabilities. These data confirm the high HLA A*02:01-binding capability of the H3.3.K27M peptide.

Then, we stimulated HLA-A*0201 donor-derived peripheral blood mononuclear cells (PBMCs) with synthetic peptide for H3.3.K27M (26-35) in vitro for several weekly cycles and evaluated for the induction of CD8+ T-cells capable of binding the HLA-A*0201-H3.3.K27M (26-35) tetramer, and found over 60% of CD8+ cells bound to the tetramer (Figure 2A). Furthermore, a subpopulation of the total CD8+ cells in the culture showed distinctively higher levels of binding to the tetramer, suggesting that these are high-affinity binders among the H3.3.K27M (26-35)-reactive T-cell populations.

### 2. Antigen-specific IFN-γ production and lytic activity of H3.3.K27M (26-35)-stimulated CD8+ T-cells.

CD8+ T cell lines that had been stimulated with the H3.3.K27M (26-35) peptide demonstrate peptide-dose-dependent increases of IFN-γ production in response to T2 target cells loaded with H3.3.K27M (26-35) compared to T2 cells loaded with the control H3.3.Non-M (26-35), cap6D peptide or nothing (Figure 2B and 3).

### 3. H3.3.K27M (26-35)-stimulated CD8+ T-cells recognize the cognate epitope endogenously expressed in HLA-A*0201 glioma cells.

We next examined whether the CD8+ T-cell line developed in response to the H3.3.K27M (26-35) peptide recognize HLA-A*0201+ human glioma cells that endogenously express and present the H3.3.K27M (26-35) epitope. We used HSID-007 (HLA-A*0201-negative but K27M+) or HSID-013 (HLA-A*0201+ and K27M+) as target glioma cells. As illustrated in Figure 2C and Figure 4, the CD8+ T-cell line responded to HSID-013 but not to HSID-007 cells based on cytotoxic T-lymphocyte (CTL) assays (Figure 2C). Furthermore, the observed response against HSID-013 cells was almost completely blocked by anti-HLA-class I blocking antibody. These data are notable because they indicate that the CD8+ T-cell lines (we have similar data for two additional cell lines; but only one is shown here) recognize the H3.3.K27M (26-25) mutant epitope endogenously expressed and presented by HLA-A*0201+ glioma cell lines.

### 4. High affinity H3.3.K27M-specific CTL clones.

We have obtained CTL clones that are reactive to the H3.3.K27M epitope (Figure 5A-B). These clones retain specificity to the H3.3. K27M epitope and we have isolated T-cell receptor (TCR) α and β chains from the clone 1H5.

### 5. Cloning of H3.3.K27M-specific T-cell receptor cDNA.

We have cloned full-length α- (SEQ ID NO:8) and β-chains (SEQ ID NO: 10) of TCRs from an H3.3.K27M-specific CD8+ T-cell clone (1H5), and constructed a lentiviral vector encoding the α- and β-chains. As the first step to confirm expression of the transgene-TCR, we transduced J.RT3-T3.5 cells, which are deficient for endogenous TCR β-chain, with the lentiviral vector and evaluated for expression of HLA-A*0201/H3.3.K27M-tetramer-reactive TCR by flow cytometry (Figure 6A; upper panels; Figure 7). More than 60% of J.RT3-T3.5 cells showed positive tetramer binding, whereas only 0.69% of non-transduced J.RT3-T3.5 cells showed the signal, indicating that the transgene TCR is expressed by both lentiviral constructs. In the same setting, we also observed that TCR-transduced cells, but not control cells upregulate CD3 expression when co-incubated with the tetramer (Figure 6A lower panels). These cells also upregulate CD69 as an indication of activation in a peptide-dose-dependent, and TCR-specific manner (Figure 6B). These data clearly indicate antigen-specific reactivity of the TCR when transduced in J.RT3-T3.5 cells.

Finally, TCR-transduced cells, but not control cells, were able to produce IL-2 when they were co-cultured with HLA-A*0201+, H3.3.K27M+ DIPG-013 cells (Figure 6C).

### 6. Absence of detectable deiminated H3 protein in cultured glioma cells

The natural process of deimination can convert histone arginine to citrulline, including the arginine residue within the H3.3.K27M epitope. Therefore, it is useful to determine whether glioma cells undergo deimination, and, if so, whether the replacement of the arginine residue within the H3.3.K27M epitope with citrulline (*i.e.* deimination) impacts the immunogenicity of the H3.3.K27M epitope.

We performed Western blot analyses to determine whether H3.3.K27M⁺ glioma cells have deiminated H3 protein using a mAb specific to deiminated H3 (abcam cat# ab19847). While neutrophils stimulated with calcium ionophore as the positive control sample showed a single band of approximately 15 kDa corresponding to the deiminated H3, none of the glioma cell lines demonstrated a similar band, strongly suggesting that glioma cell lines are not substantially deiminated, at least when cultured in vitro. The positive control sample is from neutrophils stimulated with calcium ionophore. Glioma cells evaluated are HSJD-DIPG-13, HSJD-DIPG-12, HSJD-DIPG-08, HSJD-DIPG-07, SU06, SU-D-04, T98, and U87. Each lane was loaded with 20 µg protein lysate and high quality SDS-PAGE was confirmed each time by Coomassie Blue staining. We repeated these Western blot analyses at least 4 times (4 SDS-PAGE runs) with different incubation and exposure conditions, and found a thin band with glioma cells at very high exposure conditions (not shown), suggesting that deimination may occur in cultured glioma cells at low levels.

### 7. Alanine scanning data suggests that there are no known human proteins that share the key immunogenic AA residues of the H3.3.K27M epitope

To ensure the specificity and safety of the H3.3.K27M-targeting approach, it is useful to determine key AA residues in the H3.3.K27M epitope that are responsible for the CTL reactivity. This will allow precise predictions and assessments for cross-reactivity to other epitopes derived from proteins in non-tumor normal cells.

To this end, alanine-scanning mutagenesis was used. Single alanine mutations (10 in total) were introduced at every AA residue within the H3.3.K27M decamer (10-mer) epitope. Hence, 10 synthetic peptides each containing the specific substitution with alanine were prepared (A1-A10). Using each of the synthetic peptides with alanine-substitutions, we evaluated whether the substitution alters the stability of peptide-binding to HLA-A*0201 **(****Figure 8A****)**. When the binding is diminished by the substitution compared with the natural H3.3.K27M epitope ("Mut H3.3." in **Figure 8A-B**), it indicates that the substituted residue was critical for HLA-A*0201-binding. We also evaluated whether the H3.3.K27M-specific TCR recognizes the altered epitopes presented on T2 cells. To this end, we evaluated IL-2 production as the readout for the activation of TCR-transduced J.RT3-T3.5 cells when co-cultured with T2 cells loaded with each of the altered peptides **(****Figure 8B**). AA substitutions that diminish IL-2 production (which was seen with the Mut H3.3 peptide) tell us critical AA for recognition by the TCR. Both binding- (**Figure 8A**) and function (*i.e.,* IL-2)- (**Figure 8B**) based approaches consistently showed that AA at Positions 1, 2, 4, 6, 7, 8, 9 and 10 are critical for the recognition. We then carried out *in silico* search to identify naturally existing AA sequences using NCBI BLAST. We found no known human proteins that contain the same AA residues, indicating that it would be highly unlikely that immunotherapy targeting this epitope will cause unwanted off-target reactions against normal cells.

We also evaluated whether the H3.3.K27M-specific TCR is reactive to the deiminated H3.3K.27M epitope using a synthetic peptide in which the arginine residue of the H3.3.K27M epitope is replaced by citrulline (Cit H3.3) (**Figure 8A-B**). While the Cit H3.3 peptide partially retains its affinity to HLA-A*0201 (**Figure 8A**), it completely abrogates IL-2 production by TCR-transduced J.RT3-T3.5 cells, indicating that the TCR does not recognize the Cit H3.3 peptide.

A subpopulation of glioma patients bears the K27M mutation in H3.1 (a homologue of H3.3). The AA sequences encompassing the K27M mutation in H3.1 and H3.3 are similar. When compared with the H3.3.K27M epitope, the corresponding portion of H.3.1 has only one AA substitution. Hence, we evaluated whether the TCR against the H3.3 K27M cross-reacts against H3.1 K27M using a synthetic peptide designed for the putative H3.1 K27M epitope (Mut H3.1 in **Figure 8B**). The TCR failed to recognize the Mut H.3.1 epitope, suggesting that patients with the H3.1.K27M mutation but without H3.3.K27M mutation are not eligible for prospective TCR-transduced adoptive transfer therapy.

It is understood that the examples and embodiments described herein are for illustrative purposes only and that various modifications or changes in light thereof will be suggested to persons skilled in the art.

## Claims

1. A T-cell expressing one or more polypeptides comprising a T-cell receptor (TCR) or fragment thereof that binds to a peptide/major histocompatibility complex (MHC) complex, wherein the MHC is HLA-A*0201, and wherein the peptide in the peptide/MHC complex consists of 10-14 amino acids and comprises RMSAPSTGGV (SEQ ID NO:2).

2. The T-cell of claim 1, wherein:
(a) the TCR or fragment thereof is heterologous to the T-cell;
(b) expression of the TCR or fragment thereof is under the control of a heterologous promoter;
(c) the peptide consists of RMSAPSTGGV (SEQ ID NO:2); and/or
(d) R in SEQ ID NO:2 is citrullinated or not citrullinated.

3. A method of enriching for T-cells expressing a TCR that binds to a peptide/MHC complex, wherein the MHC is HLA-A*0201, wherein the peptide in the peptide/MHC complex consists of 10-14 amino acids, and wherein the peptide comprises RMSAPSTGGV (SEQ ID NO:2), the method comprising
generating a starting culture of T-cells expressing TCRs;
culturing the T-cells in the presence of the peptide to generate an enriched culture of T-cells, wherein the enriched culture is enriched for T-cells expressing TCRs that bind the peptide/MHC complex compared to the starting culture.

4. The method of claim 3:
(a) wherein the culturing comprises culturing the T-cells in the presence of IL-2, IL-4, IL-7, IL-15, or combinations thereof;
(b) further comprising sorting cells in the enriched culture for T-cells that bind the peptide/MHC complex to form a further enriched population of T-cells expressing TCRs that bind the peptide/MHC complex;
optionally wherein the sorting comprises contacting cells in the enriched culture with a fluorochrome-conjugated peptide-major histocompatibility complex (pMHC) multimer;
(c) wherein the peptide consists of RMSAPSTGGV (SEQ ID NO:2); and/or
(d) wherein R in SEQ ID NO:2 is citrullinated or not citrullinated.

5. An isolated peptide consisting of RMSAPSTGGV (SEQ ID NO:2), optionally wherein R in SEQ ID NO:2 is citrullinated or not citrullinated.

6. A composition for stimulating an immune response to replication-independent histone 3 variant H3.3, the composition comprising the peptide of claim 5, optionally further comprising an adjuvant.

7. A fluorochrome-conjugated peptide-major histocompatibility complex (pMHC) multimer, wherein the MHC is HLA-A*0201, and wherein the peptide is according to claim 5.

8. A nucleic acid encoding the peptide of claim 5, optionally wherein the nucleic acid is a plasmid or a viral vector, optionally wherein the vector is capable of delivering the nucleic acid into an antigen presenting cell (APC).

9. An antigen presenting cell (APC) comprising a heterologous peptide consisting of 10-12 amino acids, wherein the peptide comprises RMSAPSTGGV (SEQ ID NO:2).

10. The APC of claim 9, wherein:
(a) the peptide consists of RMSAPSTGGV (SEQ ID NO:2);
(b) R in SEQ ID NO:2 is citrullinated or not citrullinated;
(c) the APC presents the heterologous peptide on the surface of the cell; and/or
(d) the APC comprises a heterologous expression cassette comprising a promoter operably linked to a polynucleotide encoding the peptide.

11. An APC of claim 9 or 10 for use in a method of treating glioma in a human individual, the method comprising,
administering a sufficient amount of the APC to the human individual.

12. The APC for use according to claim 11, wherein:
(a) the APC is from the individual (autologous);
(b) an immune response in the human individual to replication-independent histone 3 variant H3.3 is induced, optionally wherein the immune response is a cytotoxic T-cell response;
(c) the human individual has a glioma selected from glioblastoma (GBM) and diffuse intrinsic pontine gliomas (DIPG); and/or
(d) the individual carries an HLA-A*0201 allele.

13. A composition comprising a peptide consisting of 10-12 amino acids, wherein the peptide comprises RMSAPSTGGV (SEQ ID NO:2), for use in a method of treating glioma in a human individual, the method comprising administering a sufficient amount of the composition to the human individual.

14. The composition for use according to claim 13:
(a) wherein the peptide consists of RMSAPSTGGV (SEQ ID NO:2);
(b) further comprising an adjuvant; and/or
(c) wherein R in SEQ ID NO:2 is citrullinated or not citrullinated.

15. The composition for use according to claim 13 or 14, wherein the composition comprises an adjuvant selected from the group consisting of polyICLC and Bacillus Calmette-Guerin (BCG) vaccine, optionally wherein:
(a) an immune response in the human individual to histone 3 variant H3.3 is induced, optionally wherein the immune response is a cytotoxic T-cell response;
(b) the human individual has a glioma selected from GBM and DIPG; and/or
(c) the individual carries an HLA-A*0201 allele.

## Patentansprüche

1. T-Zelle, die ein oder mehrere Polypeptide exprimiert, umfassend einen T-Zellrezeptor (TCR) oder ein Fragment davon, der/das an einen Peptid/Haupthistokompatibilitätskomplex (MHC) -Komplex bindet, wobei der MHC HLA-A*0201 ist und wobei das Peptid im Peptid/MHC-Komplex aus 10-14 Aminosäuren besteht und RMSAPSTGGV (SEQ ID NO: 2) umfasst.

2. T-Zelle nach Anspruch 1, wobei:
(a) der TCR oder das Fragment davon heterolog zu der T-Zelle ist;
(b) Expression des TCR oder Fragments davon unter der Kontrolle eines heterologen Promotors steht;
(c) das Peptid aus RMSAPSTGGV (SEQ ID NO: 2) besteht; und/oder
(d) R in SEQ ID NO: 2 citrulliniert oder nicht citrulliniert ist.

3. Verfahren der Anreicherung für T-Zellen, die einen TCR exprimieren, der an einen Peptid/MHC-Komplex bindet, wobei der MHC HLA-A*0201 ist, wobei das Peptid im Peptid/MHC-Komplex aus 10-14 Aminosäuren besteht und wobei das Peptid RMSAPSTGGV (SEQ ID NO: 2) umfasst, wobei das Verfahren Folgendes umfasst
Erzeugen einer Startkultur von T-Zellen, die TCR exprimieren;
Kultivieren der T-Zellen im Vorhandensein des Peptids, um eine angereicherte Kultur von T-Zellen zu erzeugen, wobei die angereicherte Kultur für T-Zellen angereichert ist, die TCR exprimieren, die den Peptid-MHC-Komplex binden, verglichen mit der Startkultur.

4. Verfahren nach Anspruch 3:
(a) wobei das Kultivieren das Kultivieren der T-Zellen im Vorhandensein von IL-2, IL-4, IL-7, IL-15 oder Kombinationen davon umfasst;
(b) ferner umfassend das Sortieren von Zellen in der angereicherten Kultur nach T-Zellen, die den Peptid/MHC-Komplex binden, um eine weitere angereicherte Population von T-Zellen zu bilden, die TCR exprimieren, die den Peptid/MHC-Komplex binden;
optional wobei das Sortieren das Kontaktieren von Zellen in der angereicherten Kultur mit einem fluorochromkonjugierten Peptid/Haupthistokompatibilitätskomplex (pMHC) - Multimer umfasst;
(c) wobei das Peptid aus RMSAPSTGGV (SEQ ID NO: 2) besteht; und/oder
(d) wobei R in SEQ ID NO: 2 citrulliniert oder nicht citrulliniert ist.

5. Isoliertes Peptid, das aus RMSAPSTGGV (SEQ ID NO: 2) besteht, wobei optional R in SEQ ID NO: 2 citrulliniert oder nicht citrulliniert ist.

6. Zusammensetzung zum Stimulieren einer Immunantwort auf replikationsunabhängige Histon-3-Variante H3.3, wobei die Zusammensetzung das Peptid nach Anspruch 5 umfasst, optional ferner umfassend einen Hilfsstoff.

7. Fluorochromkonjugiertes Peptid/Haupthistokompatibilitätskomplex (pMHC)-Multimer wobei der MHC HLA-A*0201 ist und wobei das Peptid nach Anspruch 5 ist.

8. Nukleinsäure, die das Peptid nach Anspruch 5 kodiert, wobei optional die Nukleinsäure ein Plasmid oder ein viraler Vektor ist, wobei optional der Vektor in der Lage ist, die Nukleinsäure in eine antigenpräsentierende Zelle (APC) zuzuführen.

9. Antigenpräsentierende Zelle (APC), umfassend ein heterologes Peptid, das aus 10-12 Aminosäuren besteht, wobei das Peptid RMSAPSTGGV (SEQ ID NO: 2) umfasst.

10. APC nach Anspruch 9, wobei:
(a) das Peptid aus RMSAPSTGGV (SEQ ID NO: 2) besteht;
(b) R in SEQ ID NO: 2 citrulliniert oder nicht citrulliniert ist;
(c) die APC das heterologe Peptid auf der Oberfläche der Zelle enthält; und/oder
(d) die APC eine heterologe Expressionskassette umfasst, die einen Promotor umfasst, der mit einem Polynukleotid, das das Peptid kodiert, funktionell verknüpft ist.

11. APC nach Anspruch 9 oder 10 zur Verwendung in einem Verfahren des Behandelns von Gliom in einem menschlichen Individuum, wobei das Verfahren Folgendes umfasst Verabreichen einer ausreichenden Menge der APC an das menschliche Individuum.

12. APC zur Verwendung nach Anspruch 11, wobei:
(a) die APC aus dem Individuum stammt (autolog);
(b) eine Immunantwort im menschlichen Individuum auf replikationsunabhängige Histon-3-Variante H3.3 induziert ist, wobei optional die Immunantwort eine cytotoxische T-Zellen-Antwort ist;
(c) das menschliche Individuum ein Gliom hat, ausgewählt aus Glioblastom (GBM) und diffusem intrinsischem Ponsgliom (DIPG); und/oder
(d) das Individuum ein HLA-A*-0201-Allel trägt.

13. Zusammensetzung, umfassend ein Peptid, das aus 10-12 Aminosäuren besteht, wobei das Peptid RMSAPSTGGV (SEQ ID NO: 2) umfasst, zur Verwendung in einem Verfahren des Behandelns von Gliom in einem menschlichen Individuum, wobei das Verfahren das Verabreichen einer ausreichenden Menge der Zusammensetzung an das menschliche Individuum umfasst.

14. Zusammensetzung zur Verwendung nach Anspruch 13:
(a) wobei das Peptid aus RMSAPSTGGV (SEQ ID NO: 2) besteht;
(b) ferner umfassend einen Hilfsstoff; und/oder
(c) wobei R in SEQ ID NO: 2 citrulliniert oder nicht citrulliniert ist.

15. Zusammensetzung zur Verwendung nach Anspruch 13 oder 14, wobei die Zusammensetzung einen Hilfsstoff umfasst, ausgewählt aus der Gruppe bestehend aus polyICLC und Bacillus Calmette-Guérin (BCG)-Impfstoff, wobei optional:
(a) eine Immunantwort im menschlichen Individuum auf Histon-3-Variante H3.3 induziert ist, wobei optional die Immunantwort eine cytotoxische T-Zellen-Antwort ist;
(b) das menschliche Individuum ein Gliom hat, ausgewählt aus GBM und DIPG; und/oder
(c) das Individuum ein HLA-A*-0201-Allel trägt.

## Revendications

1. Cellule T exprimant un ou plusieurs polypeptides comprenant un récepteur de cellule T (TCR) ou un fragment de celui-ci qui se lie à un complexe peptidique / complexe majeur d'histocompatibilité (CMH), dans laquelle le CMH est HLA-A*0201, et dans laquelle le peptide dans le complexe peptidique / CMH se compose de 10 à 14 acides aminés et comprend RMSAPSTGGV (SEQ ID NO: 2).

2. Cellule T selon la revendication 1, dans laquelle :
(a) le TCR ou un fragment de celui-ci est hétérologue par rapport à la cellule T ;
(b) l'expression du TCR ou d'un fragment de celui-ci est sous le contrôle d'un promoteur hétérologue ;
(c) le peptide est constitué par RMSAPSTGGV (SEQ ID NO: 2) ; et / ou
(d) R dans SEQ ID NO: 2 est citrulliné ou non citrulliné.

3. Procédé d'enrichissement en cellules T exprimant un TCR qui se lie à un complexe peptidique / CMH, dans lequel le CMH est HLA-A*0201, dans lequel le peptide dans le complexe peptidique / CMH se compose de 10 à 14 acides aminés, et dans lequel le peptide comprend RMSAPSTGGV (SEQ ID NO: 2), le procédé comprenant
la génération d'une culture de départ de cellules T exprimant les TCR ;
la culture des cellules T en présence du peptide pour générer une culture enrichie de cellules T, dans lequel la culture enrichie est enrichie pour les cellules T exprimant les TCR qui se lient au complexe peptidique / CMH par rapport à la culture de départ.

4. Procédé selon la revendication 3 :
(a) dans lequel la culture comprend la culture des cellules T en présence d'IL-2, d'IL-4, d'IL-7, d'IL-15 ou de leurs combinaisons ;
(b) comprenant en outre le tri des cellules dans la culture enrichie pour les cellules T qui se lient au complexe peptidique / CMH pour former une population encore enrichie de cellules T exprimant des TCR qui se lient au complexe peptidique / CMH ;
éventuellement dans lequel le tri comprend la mise en contact de cellules dans la culture enrichie avec un multimère peptidique conjugué au fluorochrome - complexe majeur d'histocompatibilité (pCMH);
(c) dans lequel le peptide est constitué par RMSAPSTGGV (SEQ ID NO: 2) ; et / ou
(d) dans lequel R dans SEQ ID NO: 2 est citrulliné ou non citrulliné.

5. Peptide isolé constitué par RMSAPSTGGV (SEQ ID NO: 2), éventuellement dans lequel R dans SEQ ID NO: 2 est citrulliné ou non citrulliné.

6. Composition pour stimuler une réponse immunitaire au variant H3.3 d'histone 3 indépendant de la réplication, la composition comprenant le peptide selon la revendication 5, comprenant en outre éventuellement un adjuvant.

7. Multimère peptidique conjugué au fluorochrome - complexe majeur d'histocompatibilité (pCMH), dans lequel le CMH est HLA-A*0201, et dans lequel le peptide est selon la revendication 5.

8. Acide nucléique codant pour le peptide selon la revendication 5, éventuellement dans lequel l'acide nucléique est un plasmide ou un vecteur viral, éventuellement dans lequel le vecteur est capable de délivrer l'acide nucléique dans une cellule présentatrice d'antigène (CPA).

9. Cellule présentatrice d'antigène (CPA) comprenant un peptide hétérologue constitué de 10 à 12 acides aminés, dans laquelle le peptide comprend RMSAPSTGGV (SEQ ID NO: 2).

10. CPA selon la revendication 9, dans laquelle :
(a) le peptide est constitué par RMSAPSTGGV (SEQ ID NO: 2) ;
(b) R dans SEQ ID NO: 2 est citrulliné ou non citrulliné ;
(c) la CPA présente le peptide hétérologue à la surface de la cellule ; et / ou
(d) la CPA comprend une cassette d'expression hétérologue comprenant un promoteur lié de manière fonctionnelle à un polynucléotide codant pour le peptide.

11. CPA selon la revendication 9 ou 10 à utiliser dans un procédé de traitement du gliome chez un individu humain, le procédé comprenant,
l'administration d'une quantité suffisante de CPA à l'individu humain.

12. CPA à utiliser selon la revendication 11, dans laquelle :
(a) la CPA provient de l'individu (autologue) ;
(b) une réponse immunitaire chez l'individu humain au variant H3.3 d'histone 3 indépendant de la réplication est induite, éventuellement dans laquelle la réponse immunitaire est une réponse de cellule T cytotoxique ;
(c) l'individu humain a un gliome choisi parmi le glioblastome (GBM) et les gliomes infiltrants du tronc cérébral (GITC) ; et / ou
(d) l'individu porte un allèle HLA-A*0201.

13. Composition comprenant un peptide constitué de 10 à 12 acides aminés, dans laquelle le peptide comprend RMSAPSTGGV (SEQ ID NO: 2), à utiliser dans un procédé de traitement du gliome chez un individu humain, le procédé comprenant l'administration d'une quantité suffisante de composition à l'individu humain.

14. Composition à utiliser selon la revendication 13 :
(a) dans laquelle le peptide est constitué par RMSAPSTGGV (SEQ ID NO: 2) ;
(b) comprenant en outre un adjuvant ; et / ou
(c) dans laquelle R dans SEQ ID NO: 2 est citrulliné ou non citrulliné.

15. Composition à utiliser selon la revendication 13 ou 14, dans laquelle la composition comprend un adjuvant choisi dans le groupe constitué par le vaccin polyICLC et Bacillus Calmette-Guérin (BCG), éventuellement dans laquelle :
(a) une réponse immunitaire chez l'individu humain au variant H3.3 d'histone 3 est induite, éventuellement dans laquelle la réponse immunitaire est une réponse de cellules T cytotoxiques ;
(b) l'individu humain a un gliome choisi parmi GBM et GITC ; et / ou
(c) l'individu porte un allèle HLA-A*0201.
